# EUROPEAN PATENT APPLICATION

(11) **EP 2 354 254 A1**
(43) Date of publication of application: **10.08.2011**
(21) Application number: 10195317.2
(22) Date of filing: 29.08.2007
(51) Int. Cl.: C12Q 1/68, G01N 33/50, G01N 33/574

(54) **Biomarkers for assessing response to C-met treatment**

(30) Priority: 06.09.2006 US 842583 P
(62) Divisional of application: 07841509.8
(71) Applicant: Ortho-McNeil Pharmaceutical, Inc., Raritan, NJ 08869 (US)
(72) Inventor: Connors, Richard W., Harleysville, PA 19438 (US)
(74) Representative: Warner, James Alexander

(57) **Abstract**

Biomarkers that correlate to treatment with drugs that inhibit c-met are disclosed. These biomarkers have been shown to have utility in assessing response to the compounds. The expression level of the biomarkers is reduced upon treatment with c-met inhibitor compounds, thus indicating that these biomarkers are involved in c-met activity.

## Description

### CROSS-REFERENCE TO RELATED APPLICATIONS

This application claims priority to Application No. 60/842,583 filed on September 6, 2006.

### FIELD OF THE INVENTION

The present invention relates generally to the field of pharmacogenomics, and more specifically to new and alternative materials, methods and procedures to determine drug sensitivity in patients, including in patients with cancer. This invention aids in treating diseases and disorders based on patient response at a molecular level.

### BACKGROUND OF THE INVENTION

Protein kinases are enzymatic components of signal transduction pathways which catalyze transfer of the terminal phosphate from ATP to the hydroxy group of tyrosine, serine and/or threonine residues of proteins. Thus, compounds which inhibit protein kinase functions are valuable tools for assessing the physiological consequences of protein kinase activation. The overexpression or inappropriate expression of normal or mutant protein kinases in mammals has been a topic of extensive study and has been demonstrated to play a significant role in the development of many diseases, including diabetes, psoriasis, restenosis, ocular disease, schizophrenia, rheumatoid arthritis, atherosclerosis, cardiovascular disease and cancer. The cardiotonic benefits of kinase inhibition has also been studied. In sum, inhibitors of protein kinases have particular utility in the treatment of human and animal disease.

The hepatocyte growth factor (HGF) (also known as scatter factor) receptor, c-met, is a receptor tyrosine kinase which regulates cell proliferation, morphogenesis, and motility. The c-met gene is translated into a 170 kD protein which is processed into a cell surface receptor composed of a 140kD β-transmembrane subunit and 50 kD glycosylated extracellular α-subunit.

Mutations in c-met, over-expression of c-met and/or HGF/SF, expression of c-met and HGF/SF by the same cell, and overexpression and/or aberrant c-met signaling is present in a variety of human solid tumors and is believed to participate in angiogenesis, tumor development, invasion, and metastasis.

Cell lines with uncontrolled c-met activation, for example, are both highly invasive and metastatic. A notable difference between normal and transformed cells expressing c-met receptor is that phosphorylation of the tyrosine kinase domain in tumor cells is often independent of the presence of ligand.

C-met mutations/alterations have been identified in a number of human diseases, including tumors and cancers - for instance, hereditary and sporadic human papillary renal carcinomas, breast cancer, colorectal cancer, gastric carcinoma, glioma, ovarian cancer, hepatocellular carcinoma, head and neck squamous cell carcinomas, testicular carcinoma, basal cell carcinoma, liver carcinoma, sarcoma, malignant pleural mesothelima, melanoma, multiple myeloma, osteosarcoma, pancreatic cancer, prostate cancer, synovial sarcoma, thyroid carcinoma, non-small cell lung cancer (NSCLC) and small cell lung cancer, transitional cell carcinoma of urinary bladder, testicular carcinoma, basal cell carcinoma, liver carcinoma - and leukemias, lymphomas, and myelomas-- for instance, acute lymphocytic leukemia (ALL), acute myeloid leukemia (AML), acute promyelocytic leukemia (APL), chronic lymphocytic leukemia (CLL), chronic myeloid leukemia (CML), chronic neutrophilic leukemia (CNL), acute undifferentiated leukemia (AUL), anaplastic large-cell lymphoma (ALCL), prolymphocytic leukemia (PML), juvenile myelomonocyctic leukemia (JMML), adult T-cell ALL, AML with trilineage myelodysplasia (AML/TMDS), mixed lineage leukemia (MLL), myelodysplastic syndromes (MDSs), myeloproliferative disorders (MPD), multiple myeloma, (MM), myeloid sarcoma, non-Hodgkin's lymphoma and Hodgkin's disease (also called Hodgkin's lymphoma).

For literature on the above-mentioned association of c-met with human disease, see, for example, Maulik G, Shrikhande A, Kijima T, Ma PC, Morrison PT, Salgia R., Role of the hepatocyte growth factor receptor, c-met, in oncogenesis and potential for therapeutic inhibition.Cytokine Growth Factor Rev. 2002 Feb; 13(1):41-5 9, and cites therein: Bieche, M.H. Champeme and R. Lidereau, Infrequent mutations of the MET gene in sporadic breast tumours (letter). Int. J. Cancer 82 (1999), pp. 908-910; R.L. Camp, E.B. Rimm and D.L. Rimm , Met expression is associated with poor outcome in patients with axillary lymph node negative breast carcinoma. Cancer 86 (1999), pp. 2259-2265; L. Nakopoulou, H. Gakiopoulou, A. Keramopoulos et al., c-met tyrosine kinase receptor expression is associated with abnormal beta-catenin expression and favourable prognostic factors in invasive breast carcinoma. Histopathology 36 (2000), pp. 313-325; C. Liu, M. Park and M.S. Tsao , Over-expression of c-met proto-oncogene but not epidermal growth factor receptor or c-erbB-2 in primary human colorectal carcinomas. Oncogene. 7 (1992), pp. 181-185; K. Umeki, G. Shiota and H. Kawasaki, Clinical significance of c-met oncogene alterations in human colorectal cancer. Oncology 56 (1999), pp. 314-321; H. Kuniyasu, W. Yasui, Y. Kitadai et al., Frequent amplification of the c-met gene in scirrhous type stomach cancer. Biochem. Biophys. Res. Commun. 189 (1992), pp. 227-232; H. Kuniyasu, W. Yasui, H. Yokozaki et al., Aberrant expression of c-met mRNA in human gastric carcinomas. Int. J. Cancer 55 (1993), pp. 72-75; W.S. Park, R.R. Oh, Y.S. Kim et al., Absence of mutations in the kinase domain of the Met gene and frequent expression of Met and HGF/SF protein in primary gastric carcinomas. Apmis 108 (2000), pp. 195-200; J.H. Lee, S.U. Han, H. Cho et al., A novel germ line juxtamembrane Met mutation in human gastric cancer. Oncogene 19 (2000), pp. 4947-4953; T. Moriyama, H. Kataoka, H. Tsubouchi et al., Concomitant expression of hepatocyte growth factor (HGF), HGF activator and c-met genes in human glioma cells in vitro. FEBS Lett. 372 (1995), pp. 78-82; Y.W. Moon, R.J. Weil, S.D. Pack et al., Missense mutation of the MET gene detected in human glioma. Mod. Pathol. 13 (2000), pp. 973-977; M. Di Renzo, M. Olivero, T. Martone et al., Somatic mutations of the met oncogene are selected during metastatic spread of human HNSC carcinomas. Oncogene 19 (2000), pp. 1547-1555; K. Suzuki, N. Hayashi, Y. Yamada et al., Expression of the c-met proto-oncogene in human hepatocellular carcinoma. Hepatology 20 (1994), pp. 1231-1236; W.S. Park, S.M. Dong, S.Y. Kim et al., Somatic mutations in the kinase domain of the Met/hepatocyte growth factor receptor gene in childhood hepatocellular carcinomas. Cancer Res. 59 (1999), pp. 307-310; L. Schmidt, K. Junker, G. Weirich et al., Two North American families with hereditary papillary renal carcinoma and identical novel mutations in the MET proto-oncogene. Cancer Res. 58 (1998), pp. 1719-1722; J. Fischer, G. Palmedo, R. von Knobloch et al., Duplication and over-expression of the mutant allele of the MET proto-oncogene in multiple hereditary papillary renal cell tumours. Oncogene. 17 (1998), pp. 733-739; Z. Zhuang, W.S. Park, S. Pack et al., Trisomy 7-harbouring non-random duplication of the mutant MET allele in hereditary papillary renal carcinomas. Nat Genet 20 (1998), pp. 66-69; M. Olivero, G. Valente, A. Bardelli et al., Novel mutation in the ATP-binding site of the MET oncogene tyrosine kinase in a HPRCC family. Int. J. Cancer 82 (1999), pp. 640-643; L. Schmidt, K. Junker, N. Nakaigawa et al., Novel mutations of the MET proto-oncogene in papillary renal carcinomas. Oncogene 18 (1999), pp. 2343-2350; M. Jucker, A. Gunther, G. Gradl et al., The Met/hepatocyte growth factor receptor (HGFR) gene is over-expressed in some cases of human leukemia and lymphoma. Leuk. Res. 18 (1994), pp. 7-16; E. Tolnay, C. Kuhnen, T. Wiethege et al., Hepatocyte growth factor/scatter factor and its receptor c-met are over-expressed and associated with an increased microvessel density in malignant pleural mesothelioma. J. Cancer Res. Clin. Oncol. 124 (1998), pp. 291-296; J. Klominek, B. Baskin, Z. Liu et al., Hepatocyte growth factor/scatter factor stimulates chemotaxis and growth of malignant mesothelioma cells through c-met receptor. Int. J. Cancer 76 (1998), pp. 240-249; Thirkettle, P. Harvey, P.S. Hasleton et al., Immunoreactivity for cadherins, HGF/SF, met, and erbB-2 in pleural malignant mesotheliomas. Histopathology 36 (2000), pp. 522-528; P.G. Natali, M.R. Nicotra, M.F. Di Renzo et al., Expression of the c-met/HGF receptor in human melanocytic neoplasms: demonstration of the relationship to malignant melanoma tumour progression. Br. J. Cancer 68 (1993), pp. 746-750; 0. Hjertner, M.L. Torgersen, C. Seidel et al., Hepatocyte growth factor (HGF) induces interleukin-11 secretion from osteoblasts: a possible role for HGF in myeloma-associated osteolytic bone disease. Blood 94 (1999), pp. 3883-3888; C. Liu and M.S. Tsao , In vitro and in vivo expressions of transforming growth factor-alpha and tyrosine kinase receptors in human non-small-cell lung carcinomas. Am. J. Pathol. 142 (1993), pp. 1155-1162; M. Olivero, M. Rizzo, R. Madeddu et al., Over-expression and activation of hepatocyte growth factor/scatter factor in human non-small-cell lung carcinomas. Br J. Cancer 74 (1996), pp. 1862-1868; E. Ichimura, A. Maeshima, T. Nakajima et al., Expression of c-met/HGF receptor in human non-small cell lung carcinomas in vitro and in vivo and its prognostic significance. Jpn. J. Cancer Res. 87 (1996), pp. 1063-1069; Takanami, F. Tanana, T. Hashizume et al., Hepatocyte growth factor and c-met/hepatocyte growth factor receptor in pulmonary adenocarcinomas: an evaluation of their expression as prognostic markers. Oncology 53 (1996), pp. 392-397; J.M. Siegfried, L.A. Weissfeld, J.D. Luketich et al., The clinical significance of hepatocyte growth factor for non-small cell lung cancer. Ann Thorac. Surg. 66 (1998), pp. 1915-1918; M. Tokunou, T. Niki, K. Eguchi et al., c-met expression in myofibroblasts: role in autocrine activation and prognostic significance in lung adenocarcinoma. Am J. Pathol. 158 (2001), pp. 1451-1463; R. Ferracini, M.F. Di Renzo, K. Scotlandi et al., The Met/HGF receptor is over-expressed in human osteosarcomas and is activated by either a paracrine or an autocrine circuit. Oncogene 10 (1995), pp. 739-749; M.F. Di Renzo, M. Olivero, D. Katsaros et al., Over-expression of the Met/HGF receptor in ovarian cancer. Int. J. Cancer 58(1994), pp. 658-662; H.M. Sowter, A.N. Corps and S.K. Smith , Hepatocyte growth factor (HGF) in ovarian epithelial tumour fluids stimulates the migration of ovarian carcinoma cells. Int. J. Cancer 83 (1999), pp. 476-480; M. Ebert, M. Yokoyama, H. Friess et al., Co-expression of the c-met proto-oncogene and hepatocyte growth factor in human pancreatic cancer. Cancer Res. 54 (1994), pp. 5775-5778; L.L. Pisters, P. Troncoso, H.E. Zhau et al., c-met proto-oncogene expression in benign and malignant human prostate tissues. J. Urol. 154 (1995), pp. 293-298; P.A. Humphrey, X. Zhu, R. Zarnegar et al., Hepatocyte growth factor and its receptor (c-met) in prostatic carcinoma. Am J. Pathol. 147 (1995), pp. 386-396; K. Rygaard, T. Nakamura, M. Spang-Thomsen et al., Expression of the proto-oncogenes c-met and c-kit and their ligands, hepatocyte growth factor/scatter factor and stem cell factor, in SCLC cell lines and xenografts. Br J. Cancer 67 (1993), pp. 37-46; Y. Oda, A. Sakamoto, T. Saito et al., Expression of hepatocyte growth factor (HGF)/scatter factor and its receptor c-met correlates with poor prognosis in synovial sarcoma. Hum. Pathol. 31 (2000), pp. 185-192; M.F. Di Renzo, M. Olivero, G. Serini et al., Over-expression of the c-met/HGF receptor in human thyroid carcinomas derived from the follicular epithelium. J. Endocrinol. Invest 18 (1995), pp. 134-139; K. Gohji, M. Nomi, Y. Niitani et al., Independent prognostic value of serum hepatocyte growth factor in bladder cancer. J. Clin. Oncol. 18 (2000), pp. 2963-2971.

Because of the role of aberrant HGF/SF and/or c-met signaling in the pathogenesis of various human cancers, inhibitors of c-met receptor tyrosine kinase have broad applications in the treatment of cancers in which Met activity contributes to the invasive/metastatic phenotype, including those in which c-met is not overexpressed or otherwise altered. Inhibitors of c-met also inhibit angiogenesis and therefore are believed to have utility in the treatment of diseases associated with the formation of new vasculature, such as rheumatoid arthritis and retinopathy. See, Michieli P, Mazzone M, Basilico C, Cavassa S, Sottile A, Naldini L, Comoglio PM. Targeting the tumor and its microenvironment by a dual-function decoy Met receptor. Cancer Cell. 2004 Jul;6(1):61-73.

Over-expression of c-met is also believed to be a potentially useful predictor for the prognosis of certain diseases, such as, for example, breast cancer, non-small cell lung carcinoma, pancreatic endocrine neoplasms, prostate cancer, esophageal adenocarcinoma, colorectal cancer, salivary gland carcinoma, diffuse large B-cell lymphoma and endometrial carcinoma.

See Herrera LJ, El-Hefnawy T, Queiroz de Oliveira PE, Raja S, Finkelstein S, Gooding W, Luketich JD, Godfrey TE, Hughes SJ., The HGF Receptor c-met Is Overexpressed in Esophageal Adenocarcinoma. Neoplasia. 2005 Jan;7(1):75-84; Zeng Z, Weiser MR, D'Alessio M, Grace A, Shia J, Paty PB., Immunoblot analysis of c-met expression in human colorectal cancer: overexpression is associated with advanced stage cancer. Clin Exp Metastasis. 2004;21(5):409-17; He Y, Peng Z, Pan X, Wang H, Ouyang Y. [Expression and correlation of c-met and estrogen receptor in endometrial carcinomas] Sichuan Da Xue Xue Bao Yi Xue Ban. 2003 Jan;34(1):78-9, 88 (English Abstract Only); Tsukinoki K, Yasuda M, Mori Y, Asano S, Naito H, Ota Y, Osamura RY, Watanabe Y. Hepatocyte growth factor and c-met immunoreactivity are associated with metastasis in high grade salivary gland carcinoma. Oncol Rep. 2004 Nov;12(5):1017-21; Kawano R, Ohshima K, Karube K, Yamaguchi T, Kohno S, Suzumiya J, Kikuchi M, Tamura K. Prognostic significance of hepatocyte growth factor and c-met expression in patients with diffuse large B-cell lymphoma. Br J Haematol. 2004 Nov;127(3):305-7; Lengyel E, Prechtel D, Resau JH, Gauger K, Welk A, Lindemann K, Salanti G, Richter T, Knudsen B, Vande Woude GF, Harbeck N. C-met overexpression in node-positive breast cancer identifies patients with poor clinical outcome independent of Her2/neu. Int J Cancer. 2005 Feb 10;113(4):678-82; Hansel DE, Rahman A, House M, Ashfaq R, Berg K, Yeo CJ, Maitra A. Met proto-oncogene and insulin-like growth factor binding protein 3 overexpression correlates with metastatic ability in well-differentiated pancreatic endocrine neoplasms. Clin Cancer Res. 2004 Sep 15;10(18 Pt 1):6152-8; Knudsen BS, Edlund M. Prostate cancer and the met hepatocyte growth factor receptor. Adv Cancer Res. 2004;91:31-67; D Masuya, C Huang, D Liu, T Nakashima, et al., The tumour-stromal interaction between intratumoral c-met and stromal hepatocyte growth factor associated with tumour growth and prognosis in non-small-cell lung cancer patients. British Journal of Cancer. 2004; 90:1552-1562; Ernst Lengyel, Dieter Prechtel, James H. Resau, Katja Gauger, et al. C-met overexpression in node-positive breast cancer identifies patients with poor clinical outcome independent of Her2/neu. Int. J. Cancer 2005; 113: 678-682.

A number of drugs that reduce or inhibit the activity of c-met are currently being developed. See, for example, U.S. Provisional Patent Application Serial No. 60/752,634, entitled, "TRIAZOLOPYRIDAZINES AS KINASE MODULATORS" filed December 21, 2005, and U.S. Patent Application Serial No. 11/377,077, entitled, Acylhydrazones As Kinase Modulators, filed June 13, 2006, the entire contents of which are incorporated herein by reference. New prognostic and predictive markers are needed to accurately foretell a patient's response to such drugs in the clinic. Such markers would facilitate the individualization of therapy for each patient.

The present invention is directed to the identification of biomarkers that can better predict a patient's sensitivity to treatment or therapy with drugs that reduce or inhibit c-met. The classification of patient samples can aid in diagnosis and treatment. The association of a patient's response to drug treatment with one or more specific markers can open up new opportunities for drug development in non-responding patients, or distinguish a drug's indication among other treatment choices because of higher confidence in the efficacy. Further, the pre-selection of patients who are likely to respond well to a drug or combination therapy may reduce the number of patients needed in a clinical study or accelerate the time needed to complete a clinical development program (M. Cockett et al., 2000, Current Opinion in Biotechnology, 11:602-609).

A major goal of pharmacogenomics research is to identify genetic markers that accurately predict a given patient's response to drugs in the clinic; such individualized genetic assessment may greatly facilitate personalized treatment. An approach of this nature is particularly needed in cancer treatment and therapy, where commonly used drugs are ineffective in many patients, and side effects are frequent. The ability to predict drug sensitivity in patients is particularly challenging because drug responses reflect both the properties intrinsic to the target cells and also a host's metabolic properties.

Needed in the art are new and alternative materials, methods and procedures to determine drug sensitivity in patients and which are necessary to treat diseases and disorders, particularly cancers, based on patient response at a molecular level. The present invention involves the identification of polynucleotides that correlate with drug sensitivity to drugs that reduce or inhibit c-met. The presently described identification of marker polynucleotides in cell lines assayed in vitro can be used to correlate with drug responses in vivo, and thus can be extended to clinical situations in which the same polynucleotides are used to predict responses to drugs that reduce or inhibit c-met by patients.

### SUMMARY OF THE INVENTION

The present invention describes the identification of marker polynucleotides whose expression levels are highly correlated with signaling which reflect inhibition of the c-met receptor. These polynucleotides or "markers" show utility in predicting a host's response to a drug and/or drug treatment.

It is another aspect of the invention to provide a method of determining or predicting if an individual requiring treatment for a disease state, or a cancer or tumor of a particular type will successfully respond or will not respond to a drug prior to the administration of such drug. Preferably, the drug is an inhibitor of c-met. Also in accordance with the present invention, cells from a patient tissue sample are assayed to determine their polynucleotide expression pattern prior to treatment with a c-met modulating drug. The resulting polynucleotide expression profile of the test cells before exposure to the drug is compared with the polynucleotide expression pattern of the predictor set of polynucleotides.

Success or failure of treatment with a drug can be determined based on the polynucleotide expression pattern of cells from the test tissue (test cells), e.g., a tumor or cancer biopsy, as being relatively similar to or different from the polynucleotide expression pattern of the predictor set of polynucleotides. Thus, if the test cells show a polynucleotide expression profile which corresponds to that of the predictor set of polynucleotides in the control panel of cells which are sensitive to the drug, it is highly likely or predicted that the individual's cancer or tumor will respond favorably to treatment with the drug. By contrast, if the test cells show a polynucleotide expression pattern corresponding to that of the predictor set of polynucleotides of the control panel of cells which are resistant to the drug, it is highly likely or predicted that the individual's cancer or tumor will not respond to treatment with the drug.

It is a further aspect of this invention to provide screening assays for determining if a cancer patient will be susceptible or resistant to treatment with a drug, particularly, a drug directly or indirectly involved in c-met activity or a c-met pathway.

In a more particular aspect, the present invention provides screening assays for determining if a cancer patient will be susceptible or resistant to treatment with a drug, particularly, a drug directly or indirectly involved in c-met activity or the c-met pathway.

It is another aspect of the invention to provide a method of monitoring the treatment of a patient having a disease treatable by a drug that modulates c-met. This can be accomplished by comparing the resistance or sensitivity polynucleotide expression profile of cells from a patient tissue sample, e.g., a tumor or cancer biopsy, prior to treatment with a drug that inhibits c-met activity and again following treatment with the drug. The isolated test cells from the patient's tissue sample are assayed to determine their polynucleotide expression pattern before and after exposure to a drug, such as, e.g., a c-met inhibitor. The resulting polynucleotide expression profile of the test cells before and after treatment is compared with the polynucleotide expression pattern of the predictor set and subsets of polynucleotides that have been described and shown herein to be highly expressed in the control panel of cells that are either resistant or sensitive to the drug. Thus, if a patient's response becomes one that is sensitive to treatment by a c-met inhibitor compound, based on a correlation of the expression profile of the predictor polynucleotides, the patient's treatment prognosis can be qualified as favorable and treatment can continue. Also, if after treatment with a drug, the test cells do not show a change in their polynucleotide expression profile that corresponds to the control panel of cells that are sensitive to the drug, this can serve as an indicator that the current treatment should be modified, changed, or even discontinued. Such a monitoring process can indicate success or failure of a patient's treatment with a drug, and the monitoring processes can be repeated as necessary or desired.

It is a further aspect of the invention to provide predictor polynucleotides and predictor sets of polynucleotides having both diagnostic and prognostic value in disease areas in which signaling through c-met or a c-met pathway is of importance, e.g., in cancers and tumors, in immunological disorders, conditions or dysfunctions, or in disease states in which cell signaling and/or proliferation controls are abnormal or aberrant.

It is a further aspect of the invention to provide a kit for determining or predicting drug susceptibility or resistance by a patient having a disease, including with regard to a cancer or tumor. Such kits are useful in a clinical setting for testing a patient's biopsied tumor or cancer sample, for example, to determine or predict if the patient's tumor or cancer will be resistant or sensitive to a given treatment or therapy with a drug that is directly or indirectly involved with modification, preferably, inhibition, of the activity of c-met or a cell signaling pathway involving c-met activity. Provided in the kit are one or more polynucleotides that correlate with resistance and sensitivity to c-met modulators; and, in suitable containers, the drug for use in testing cells from patient tissue specimens or patient samples; and instructions for use. In addition, kits contemplated by the present invention can include reagents or materials for the monitoring of the expression of the predictor or marker polynucleotides of the invention at the level of mRNA or protein, using other techniques and systems practiced in the art, e.g., RT-PCR assays, which employ primers designed on the basis of one or more of the predictor polynucleotides described herein, immunoassays, such as enzyme linked immunosorbent assays (ELISAs), immunoblotting, e.g., Western blots, or in situ hybridization, and the like, as further described herein.

Another aspect of the invention is to provide one or more polynucleotides among those of the predictor polynucleotides identified herein that can serve as targets for the development of drug therapies for disease treatment. Such targets can be particularly applicable to treatment of disease, such as cancers or tumors. Because these predictor polynucleotides are differentially expressed in sensitive and resistant cells, their expression pattern is correlated with the relative intrinsic sensitivity of cells to treatment with compounds that interact with and/or inhibit c-met. Accordingly, the polynucleotides highly expressed in resistant cells can serve as targets for the development of new drug therapies for those tumors which are resistant to c-met inhibitor compounds.

Yet another object of the invention is to provide antibodies, either polyclonal or monoclonal, directed against one or more of the c-met biomarker polypeptides, or peptides thereof, encoded by the predictor polynucleotides. Such antibodies can be used in a variety of ways, for example, to purify, detect, and target the c-met biomarker polypeptides of the present invention, including both in vitro and in vivo diagnostic, detection, screening, and/or therapeutic methods, and the like.

Yet another object of the invention is to provide antisense reagents, including siRNA, RNAi, and ribozyme reagents, directed against one or more of the c-met biomarker polypeptides, or peptides thereof, encoded by the predictor polynucleotides. Such antisense reagents can be used in a variety of ways, for example, to detect, to target, and inhibit the expression of the c-met biomarker polypeptides of the present invention, including both in vitro and in vivo diagnostic, detection, screening, and/or therapeutic methods, and the like

### DESCRIPTION OF THE FIGURES

FIG. 1 is a graph showing VEGF mRNA levels in PC-3 prostate carcinoma cells upon stimulation with HGF at t = 0 to 48 hours.
FIG. 2 is a graph showing CD44 mRNA levels in PC-3 prostate carcinoma cells upon stimulation with HGF at t = 0 to 48 hours.
FIG. 3 is a graph showing MMP-1 mRNA levels in PC-3 prostate carcinoma cells upon stimulation with HGF at t = 0 to 48 hours.
FIG. 4 is a graph showing fibronectin mRNA levels in PC-3 prostate carcinoma cells upon stimulation with HGF at t = 0 to 48 hours.
FIG. 5 is a bar chart showing upregulation of VEGF and CD44 with HGF in U87MG glioblastoma cells, as well as inhibition of VEGF and CD44 upregulation via c-met inhibitor 3DP-669366, at t = 4 to 24 hours.
FIG. 6 is a bar chart showing inhibition of VEGF and CD44 upregulation in PC-3, DU145 and U118 cells via c-met inhibitor 3DP-669366.
FIG. 7 is a bar chart showing dose response of 3DP-669366 on VEGF mRNA in PC-3 cells.
FIG. 8 is a bar chart showing dose response of 3DP-669366 on VEGF mRNA in DU cells.
FIG. 9 is a bar chart showing dose response of 3DP-669366 on MMP-1 mRNA in DU cells.
FIG. 10 is a bar chart showing dose response of 3DP-669366 on MMP-1 mRNA in PC-3 cells.
FIG. 11 is a bar chart showing the effect of acylhydrazones JNJ-28823184 and JNJ-38429274 on CD44 in U87MG glioblastoma cells.
FIG. 12 is a bar chart showing the effect of acylhydrazones JNJ-28823184 and JNJ-38429274 on LDLR-1 in U87MG glioblastoma cells.
FIG. 13 is a bar chart showing the effect of acylhydrazones JNJ-28823184 and JNJ-38429274 on MET in U87MG glioblastoma cells.
FIG. 14 is a bar chart showing the effect of acylhydrazones JNJ-28823184 and JNJ-38429274 on MMP-1 in U87MG glioblastoma cells.
FIG. 15 is a bar chart showing the effect of acylhydrazones JNJ-28823184 and JNJ-38429274 on osteopontin in U87MG glioblastoma cells.
FIG. 16 is a bar chart showing the effect of acylhydrazones JNJ-28823184 and JNJ-38429274 on VEGF in U87MG glioblastoma cells.
FIG. 17 is a table showing the underlying data for FIGS. 14-16.
FIG. 18 is a bar chart showing the effect of acylhydrazone JNJ-28823184 on CD44, LDLR-1, MMP-1 and VEGF in U87MG glioblastoma cells.

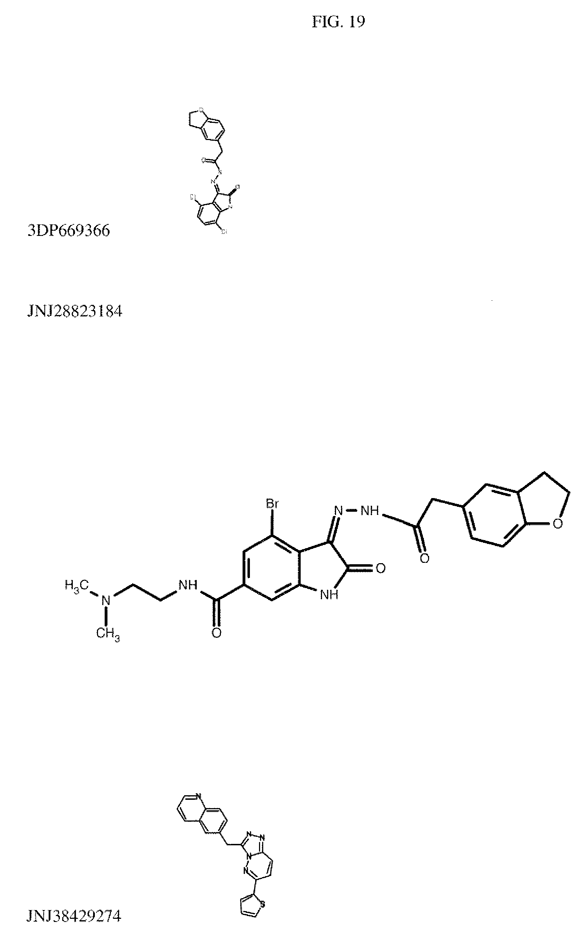

FIG. 19 is a diagram showing the structures of 3DP669366; JNJ28823124; and JNJ38429274.

### DETAILED DESCRIPTION OF THE INVENTION

All publications cited herein are hereby incorporated by reference. Unless defined otherwise, all technical and scientific terms used herein have the same meaning as commonly understood to one of ordinary skill in the art to which this invention pertains.

### Definitions

As used herein, the terms "comprising", "containing", "having" and "including" are used in their open, non-limiting sense.

A "biological sample" as used herein refers to a sample containing or consisting of cell or tissue matter, such as cells or biological fluids isolated from a subject. The "subject" can be a mammal, such as a rat, a mouse, a monkey, or a human, that has been the object of treatment, observation or experiment. Examples of biological samples include, for example, sputum, blood, blood cells (e.g., white blood cells), amniotic fluid, plasma, semen, saliva, bone marrow, tissue or fine-needle biopsy samples, urine, peritoneal fluid, pleural fluid, and cell cultures. Biological samples may also include sections of tissues such as frozen sections taken for histological purposes. A test biological sample is the biological sample that has been the object of analysis, monitoring, or observation. A control biological sample can be either a positive or a negative control for the test biological sample. Often, the control biological sample contains the same type of tissues, cells and/or biological fluids of interest as that of the test biological sample. In particular embodiments, the biological sample is a "clinical sample," which is a sample derived from a human patient.

A "cell" refers to at least one cell or a plurality of cells appropriate for the sensitivity of the detection method. The cell can be present in a cultivated cell culture. The cell can also be present in its natural environment, such as a biological tissue or fluid. Cells suitable for the present invention may be bacterial, but are preferably eukaryotic, and are most preferably mammalian.

"Nucleotide sequence" refers to the arrangement of either deoxyribonucleotide or ribonucleotide residues in a polymer in either single- or double-stranded form. Nucleic acid sequences can be composed of natural nucleotides of the following bases: thymidine, adenine, cytosine, guanine, and uracil; abbreviated T, A, C, G, and U, respectively, and/or synthetic analogs of the natural nucleotides.

An "isolated" nucleic acid molecule is one that is substantially separated from at least one of the other nucleic acid molecules present in the natural source of the nucleic acid, or is substantially free of at least one of the chemical precursors or other chemicals when the nucleic acid molecule is chemically synthesized. An "isolated" nucleic acid molecule can also be, for example, a nucleic acid molecule that is substantially free of at least one of the nucleotide sequences that naturally flank the nucleic acid molecule at its 5' and 3' ends in the genomic DNA of the organism from which the nucleic acid is derived. A nucleic acid molecule is "substantially separated from" or "substantially free of" other nucleic acid molecule(s) or other chemical(s) in preparations of the nucleic acid molecule when there is less than about 30%, 20%, 10%, or 5% (by dry weight) of the other nucleic acid molecule(s) or the other chemical(s) (also referred to herein as a "contaminating nucleic acid molecule" or a "contaminating chemical").

Isolated nucleic acid molecules include, without limitation, separate nucleic acid molecules (e.g., cDNA or genomic DNA fragments produced by PCR or restriction endonuclease treatment) independent of other sequences, as well as nucleic acid molecules that are incorporated into a vector, an autonomously replicating plasmid, a virus (e.g., a retrovirus, adenovirus, or herpes virus), or into the genomic DNA of a prokaryote or eukaryote. In addition, an isolated nucleic acid molecule can include a nucleic acid molecule that is part of a hybrid or fusion nucleic acid molecule. An isolated nucleic acid molecule can be a nucleic acid sequence that is: (i) amplified in vitro by, for example, polymerase chain reaction (PCR); (ii) synthesized by, for example, chemical synthesis; (iii) recombinantly produced by cloning; or (iv) purified, as by cleavage and electrophoretic or chromatographic separation.

The term "oligonucleotide" or "oligo" refers to a single-stranded DNA or RNA sequence of a relatively short length, for example, less than 100 residues long. For many methods, oligonucleotides of about 16-25 nucleotides in length are useful, although longer oligonucleotides of greater than about 25 nucleotides may sometimes be utilized. Some oligonucleotides can be used as "primers" for the synthesis of complimentary nucleic acid strands. For example, DNA primers can hybridize to a complimentary nucleic acid sequence to prime the synthesis of a complimentary DNA strand in reactions using DNA polymerases. Oligonucleotides are also useful for hybridization in several methods of nucleic acid detection, for example, in Northern blotting or in situ hybridization.

The terms "polypeptide," "protein," and "peptide" are used herein interchangeably to refer to amino acid chains in which the amino acid residues are linked by peptide bonds or modified peptide bonds. The amino acid chains can be of any length of greater than two amino acids. Unless otherwise specified, the terms "polypeptide," "protein," and "peptide" also encompass various modified forms thereof. Such modified forms may be naturally occurring modified forms or chemically modified forms. Examples of modified forms include, but are not limited to, glycosylated forms, phosphorylated forms, myristoylated forms, palmitoylated forms, ribosylated forms, acetylated forms, ubiquitinated forms, etc. Modifications also include intra-molecular crosslinking and covalent attachment to various moieties such as lipids, flavin, biotin, polyethylene glycol or derivatives thereof, etc. In addition, modifications may also include cyclization, branching and cross-linking. Further, amino acids other than the conventional twenty amino acids encoded by the codons of genes may also be included in a polypeptide.

An "isolated protein" is one that is substantially separated from at least one of the other proteins present in the natural source of the protein, or is substantially free of at least one of the chemical precursors or other chemicals when the protein is chemically synthesized. A protein is "substantially separated from" or "substantially free oF' other protein(s) or other chemical(s) in preparations of the protein when there is less than about 30%, 20%, 10%, or 5% (by dry weight) of the other protein(s) or the other chemical(s) (also referred to herein as a "contaminating protein" or a "contaminating chemical").

Isolated proteins can have several different physical forms. The isolated protein can exist as a full-length nascent or unprocessed polypeptide, or as a partially processed polypeptide or as a combination of processed polypeptides. The full-length nascent polypeptide can be postranslationally modified by specific proteolytic cleavage events that result in the formation of fragments of the full-length nascent polypeptide. A fragment, or physical association of fragments can have the biological activity associated with the full-length polypeptide; however, the degree of biological activity associated with individual fragments can vary.

An isolated polypeptide can be a non- naturally occurring polypeptide. For example, an "isolated polypeptide" can be a "hybrid polypeptide." An "isolated polypeptide" can also be a polypeptide derived from a naturally occurring polypeptide by additions or deletions or substitutions of amino acids. An isolated polypeptide can also be a "purified polypeptide" which is used herein to mean a specified polypeptide in a substantially homogeneous preparation substantially free of other cellular components, other polypeptides, viral materials, or culture medium, or when the polypeptide is chemically synthesized, chemical precursors or by-products associated with the chemical synthesis. A "purified polypeptide" can be obtained from natural or recombinant host cells by standard purification techniques, or by chemical synthesis, as will be apparent to skilled artisans.

"Recombinant" refers to a nucleic acid, a protein encoded by a nucleic acid, a cell, or a viral particle, that has been modified using molecular biology techniques to something other than its natural state. For example, recombinant cells can contain nucleotide sequence that is not found within the native (non-recombinant) form of the cell or can express native genes that are otherwise abnormally, under- expressed, or not expressed at all. Recombinant cells can also contain genes found in the native form of the cell wherein the genes are modified and re-introduced into the cell by artificial means. The term also encompasses cells that contain an endogenous nucleic acid that has been modified without removing the nucleic acid from the cell; such modifications include those obtained, for example, by gene replacement, and site-specific mutation.

A "recombinant host cell" is a cell that has had introduced into it a recombinant DNA sequence. Recombinant DNA sequence can be introduced into host cells using any suitable method including, for example, electroporation, calcium phosphate precipitation, microinjection, transformation, biolistics and viral infection. Recombinant DNA may or may not be integrated (covalently linked) into chromosomal DNA making up the genome of the cell. For example, the recombinant DNA can be maintained on an episomal element, such as a plasmid. Alternatively, with respect to a stably transformed or transfected cell, the recombinant DNA has become integrated into the chromosome so that it is inherited by daughter cells through chromosome replication. This stability is demonstrated by the ability of the stably transformed or transfected cell to establish cell lines or clones comprised of a population of daughter cells containing the exogenous DNA. Recombinant host cells may be prokaryotic or eukaryotic, including bacteria such as E. coli, fungal cells such as yeast, mammalian cells such as cell lines of human, bovine, porcine, monkey and rodent origin, and insect cells such as Drosophila- and silkworm-derived cell lines. It is further understood that the term "recombinant host cell" refers not only to the particular subject cell, but also to the progeny or potential progeny of such a cell. Because certain modifications can occur in succeeding generations due to either mutation or environmental influences, such progeny may not, in fact, be identical to the parent cell, but are still included within the scope of the term as used herein.

"Sequence" means the linear order in which monomers occur in a polymer, for example, the order of amino acids in a polypeptide or the order of nucleotides in a polynucleotide.

"Sequence identity or similarity", as known in the art, is the relationship between two or more polypeptide sequences or two or more polynucleotide sequences, as determined by comparing the sequences. As used herein, "identity", in the context of the relationship between two or more nucleic acid sequences or two or more polypeptide sequences, refers to the percentage of nucleotide or amino acid residues, respectively, that are the same when the sequences are optimally aligned and analyzed. For purposes of comparing a queried sequence against, for example, the amino acid sequence SEQ ID NO:2, the queried sequence is optimally aligned with SEQ ID NO: 2 and the best local alignment over the entire length of SEQ ID NO:2 is obtained.

Analysis can be carried out manually or using sequence comparison algorithms. For sequence comparison, typically one sequence acts as a reference sequence, to which a queried sequence is compared. When using a sequence comparison algorithm, test and reference sequences are input into a computer, sub-sequence coordinates are designated, if necessary, and sequence algorithm program parameters are designated.

Optimal alignment of sequences for comparison can be conducted, for example, by using the homology alignment algorithm of Needleman & Wunsch, J Mol. Biol., 48:443 (1970). Software for performing Needleman & Wunsch analyses is publicly available through the Institut Pasteur (France) Biological Software website: http://bioweb.pasteur.fr/seqanal/ interfaces/needle.html. The NEEDLE program uses the Needleman-Wunsch global alignment algorithm to find the optimum alignment (including gaps) of two sequences when considering their entire length. The identity is calculated along with the percentage of identical matches between the two sequences over the reported aligned region, including any gaps in the length. Similarity scores are also provided wherein the similarity is calculated as the percentage of matches between the two sequences over the reported aligned region, including any gaps in the length. Standard comparisons utilize the EBLOSUM62 matrix for protein sequences and the EDNAFULL matrix for nucleotide sequences. The gap open penalty is the score taken away when a gap is created; the default setting using the gap open penalty is 10.0. For gap extension, a penalty is added to the standard gap penalty for each base or residue in the gap; the default setting is 0.5.

Hybridization can also be used as a test to indicate that two polynucleotides are substantially identical to each other. Polynucleotides that share a high degree of identity will hybridize to each other under stringent hybridization conditions. "Stringent hybridization conditions" has the meaning known in the art, as described in Sambrook et al., Molecular Cloning: A Laboratory Manual, Second Edition, Cold Spring Harbor Laboratory, Cold Spring Harbor, New York, (1989). An exemplary stringent hybridization condition comprises hybridization in 6x sodium chloride/sodium citrate (SSC) at about 45 °C , followed by one or more washes in 0.2x SSC and 0.1% SDS at 50 - 65 °C , depending upon the length over which the hybridizing polynucleotides share complementarity.

"Vector" refers to a nucleic acid molecule into which a heterologous nucleic acid can be or is inserted. Some vectors can be introduced into a host cell allowing for replication of the vector or for expression of a protein that is encoded by the vector or construct. Vectors typically have selectable markers, for example, genes that encode proteins allowing for drug resistance, origins of replication sequences, and multiple cloning sites that allow for insertion of a heterologous sequence. Vectors are typically plasmid-based and are designated by a lower case "p" followed by a combination of letters and/or numbers. Starting plasmids disclosed herein are either commercially available, publicly available on an unrestricted basis, or can be constructed from available plasmids by application of procedures known in the art. Many plasmids and other cloning and expression vectors that can be used in accordance with the present invention are well-known and readily available to those of skill in the art. Moreover, those of skill readily may construct any number of other plasmids suitable for use in the invention. The properties, construction and use of such plasmids, as well as other vectors, in the present invention will be readily apparent to those of skill from the present disclosure.

The present invention describes the identification of polynucleotides that correlate with drug sensitivity or resistance of untreated cell lines to determine or predict sensitivity of the cells to a drug that reduces or inhibits c-met. These polynucleotides, called marker or predictor polynucleotides herein, can be employed for predicting drug response.

Such marker polynucleotides serve as useful molecular tools for predicting a response to drugs that affect c-met activity via direct or indirect inhibition or antagonism of the c-met function or activity.

Such a predictor set of cellular polynucleotide expression patterns correlating with sensitivity or resistance of cells following exposure of the cells to a drug, or a combination of drugs, provides a useful tool for screening a cancer, tumor, or patient test sample before treatment with the drug or drug combination. The screening technique allows prediction of cells of a disease, e.g., cancer, tumor, or test sample exposed to a drug, or a combination of drugs, based on the polynucleotide expression results of the predictor set, as to whether or not the disease, e.g., cancer, tumor, or test sample, and hence a patient harboring the disease, e.g., cancer and/or tumor, will or will not respond to treatment with the drug or drug combination. In addition, the predictor polynucleotides or predictor polynucleotide set can also be utilized as described herein for monitoring the progress of disease treatment or therapy in those patients undergoing treatment involving c-met, e.g., c-met inhibitor compound.

In another related embodiment, the present invention includes a method of predicting, prognosing, diagnosing, and/or determining whether an individual requiring drug therapy for a disease state will or will not respond to treatment prior to administration of treatment. The treatment or therapy preferably involves a c-met modulating drug, for example, an inhibitor of c-met kinase activity. In accordance with this embodiment, cells from a patient's tissue sample, e.g., a tumor or cancer biopsy, are assayed to determine their polynucleotide expression pattern prior to treatment with the c-met modulating drug. The resulting polynucleotide expression profile of the test cells before exposure to the drug is compared with that of one or more of the predictor subsets of polynucleotides.

Success or failure of treatment of a patient's disease, including cancer or tumor, with the drug can be determined based on the polynucleotide expression pattern of the patient's cells being tested, compared with the polynucleotide expression pattern of the predictor polynucleotides in the resistant or sensitive panel that have been exposed to the drug and subjected to the predictor polynucleotide analysis detailed herein. Thus, if following exposure to the drug, the test cells show a polynucleotide expression pattern corresponding to that of the predictor polynucleotide set of the control panel of cells that is sensitive to the drug, it is highly likely or predicted that the individual's disease, cancer or tumor, will respond favorably to treatment with the drug. By contrast, if, after drug exposure, the test cells show a polynucleotide expression pattern corresponding to that of the predictor polynucleotide set of the control panel of cells that is resistant to the drug, it is highly likely or predicted that the individual's disease, cancer or tumor will not respond to treatment with the drug.

In a related embodiment, screening assays are provided for determining if a patient's disease, cancer or tumor is or will be susceptible or resistant to treatment with a drug, particularly, a drug directly or indirectly involved in c-met activity or a c-met pathway.

Also provided by the present invention are monitoring assays to monitor the progress of drug treatment involving drugs that interact with or inhibit c-met activity. Such in vitro assays are capable of monitoring the treatment of a patient having a disease treatable by a drug that modulates or interacts with c-met by comparing the resistance or sensitivity polynucleotide expression pattern of cells from a patient tissue sample, e.g., a tumor or cancer biopsy, prior to treatment with a drug that inhibits c-met activity and again following treatment with the drug with the expression pattern of one or more of the predictor polynucleotide sets described, or combinations thereof. Isolated cells from the patient are assayed to determine their polynucleotide expression pattern before and after exposure to a drug, preferably a c-met inhibitor, to determine if a change of the polynucleotide expression profile has occurred so as to warrant treatment with another drug, or whether current treatment should be discontinued. The resulting polynucleotide expression profile of the cells tested before and after treatment is compared with the polynucleotide expression pattern of the predictor set of polynucleotides that have been described and shown herein to be highly expressed in cells that are either resistant or sensitive to the drug. Alternatively, a patient's progress related to drug treatment or therapy can be monitored by obtaining a polynucleotide expression profile as described above, only after the patient has undergone treatment with a given drug. In this way, there is no need to test a patient sample prior to treatment with the drug.

Such a monitoring process can indicate success or failure of a patient's treatment with a drug based on the polynucleotide expression pattern of the cells isolated from the patient's sample, e.g., a tumor or cancer biopsy, as being relatively the same as or different from the polynucleotide expression pattern of the predictor polynucleotide set of the resistant or sensitive control panel of cells that have been exposed to the drug and assessed for their polynucleotide expression profile following exposure. Thus, if, after treatment with a drug, the test cells show a change in their polynucleotide expression profile from that seen prior to treatment to one which corresponds to that of the predictor polynucleotide set of the control panel of cells that are resistant to the drug, it can serve as an indicator that the current treatment should be modified, changed, or even discontinued. Also, should a patient's response be one that shows sensitivity to treatment with a c-met inhibitor, based on correlation of the expression profile of the predictor polynucleotides of cells showing drug sensitivity with the polynucleotide expression profile from cells from a patient undergoing treatment, the patient's treatment prognosis can be qualified as favorable and treatment can continue. Further, if a patient has not been tested prior to drug treatment, the results obtained after treatment can be used to determine the resistance or sensitivity of the cells to the drug based on the polynucleotide expression profile compared with the predictor polynucleotide set.

In a related embodiment, the present invention embraces a method of monitoring the treatment of a patient having a disease treatable by a drug that modulates c-met. For these assays, test cells from the patient are assayed to determine their polynucleotide expression pattern before and after exposure to a c-met inhibitor drug. The resulting polynucleotide expression profile of the cells tested before and after treatment is compared with the polynucleotide expression pattern of the predictor set of polynucleotides that have been described and shown herein to be highly expressed in cells that are either resistant or sensitive to the drug. Thus, if a patient's response is or becomes one that is sensitive to treatment by a c-met inhibitor, based on correlation of the expression profile of the predictor polynucleotides, the patient's treatment prognosis can be qualified as favorable and treatment can continue. Also, if after treatment with a drug, the test cells do not exhibit a change in their polynucleotide expression profile to a profile that corresponds to that of the control panel of cells that are sensitive to the drug, this serves as an indicator that the current treatment should be modified, changed, or even discontinued. Such monitoring processes can be repeated as necessary or desired and can indicate success or failure of a patient's treatment with a drug, based on the polynucleotide expression pattern of the cells isolated from the patient's sample. The monitoring of a patient's response to a given drug treatment can also involve testing the patient's cells in the assay as described, only after treatment, rather than before and after treatment, with drug.

In a preferred embodiment, the present invention embraces a method of monitoring the treatment of a patient having a disease treatable by a drug that modulates c-met. The test cells from the patient are assayed to determine their polynucleotide expression pattern before and after exposure to a c-met inhibitor compound. The resulting polynucleotide expression profile of the cells tested before and after treatment is compared with the polynucleotide expression pattern of the predictor set of polynucleotides that have been described and shown herein to be highly expressed in cells that are either resistant or sensitive to the drug. Thus, if a patient's response is or becomes one that is sensitive to treatment by a c-met inhibitor compound, based on correlation of the expression profile of the predictor polynucleotides, the patient's treatment prognosis can be qualified as favorable and treatment can continue. Also, if after treatment with a drug, the test cells do not exhibit a change in their polynucleotide expression profile to a profile that corresponds to that of the control panel of cells that are sensitive to the drug, this serves as an indicator that the current treatment should be modified, changed, or even discontinued. Such monitoring processes can be repeated as necessary or desired and can indicate success or failure of a patient's treatment with a drug, based on the polynucleotide expression pattern of the cells isolated from the patient's sample. The monitoring of a patient's response to a given drug treatment can also involve testing the patient's cells in the assay as described only after treatment, rather than before and after treatment, with drug.

In another embodiment, the present invention encompasses a method of classifying whether a biological system, preferably cells from a tissue, organ, tumor or cancer of an afflicted individual, will be resistant or sensitive to a drug that modulates the system. In a preferred aspect of this invention, the sensitivity or resistance of cells, e.g., those obtained from a tumor or cancer, to a c-met inhibitor is determined. Inhibitors can include those drugs that inhibit, either directly or indirectly, c-met. According to the method, a resistance/sensitivity profile of the cells after exposure to the c-met inhibitor drug can be determined via polynucleotide expression profiling protocols set forth herein. Such resistance/sensitivity profile of the cells reflects an IC₅₀ value of the cells to the drugs as determined using a suitable assay, such as an in vitro cytotoxicity assay. A procedure of this sort can be performed using a variety of cell types and drugs that interact with the c-met or affect its activity in the signaling pathway of the c-met.

Preferably, the predictor polynucleotide sets are common for predicting sensitivity among more than one c-met modulator, e.g. a c-met inhibitor. In accordance with this aspect of the invention, the oligonucleotide sequences or cDNA sequences include any of the predictor polynucleotides or polynucleotide combinations as described herein, which are highly expressed in resistant or sensitive cells, and are contained on a microarray, e.g., a oligonucleotide microarray or cDNA microarray in association with, or introduced onto, any supporting material, such as glass slides, nylon membrane filters, glass or polymer beads, chips, plates, or other types of suitable substrate material.

Cellular nucleic acid, e.g., RNA, is isolated either from cells undergoing testing after exposure to a drug that interacts with c-met as described herein, or its signaling pathway, or from cells being tested to obtain an initial determination or prediction of the cells' sensitivity to the drug, and, ultimately, a prediction of treatment outcome with the drug. The isolated nucleic acid is appropriately labeled and applied to one or more of the specialized microarrays. The resulting pattern of polynucleotide expression on the specialized microarray is analyzed as described herein and known in the art. A pattern of polynucleotide expression correlating with either sensitivity or resistance to the drug or compound is able to be determined.

In accordance with the specialized microarray embodiment of this invention, the microarray contains the polynucleotides of one or more of the predictor polynucleotide set(s) or subset(s), or a combination thereof. If the nucleic acid target isolated from test cells, such as tumor or cancer cells shows a high level of detectable binding to the polynucleotides of the predictor set for drug sensitivity relative to control, then it can be predicted that a patient's cells will respond to the drug, or a series of drugs, and that the patient's response to the drug, or a series of drugs, will be favorable.

Such a result predicts that the cells of, e.g., a tumor or cancer, are good candidates for the successful treatment or therapy utilizing the drug, or series of drugs. Alternatively, if the nucleic acid target isolated from test cells shows a high level of detectable binding to the polynucleotides of the predictor set for drug resistance, relative to control, then it can be predicted that a patient is likely not to respond to the drug, or a series of drugs, and that the patient's response to the drug, or a series of drugs, is not likely to be favorable. Such a result predicts that the cells of a tumor or cancer are not good candidates for treatment or therapy utilizing the drug, or series of drugs.

The utilization of microarray technology is known and practiced in the art. Briefly, to determine polynucleotide expression using microarray technology, polynucleotides, e.g., RNA, DNA, cDNA, preferably RNA, are isolated from a biological sample, e.g., cells, as described herein for breast cells, using procedures and techniques that are practiced in the art. The isolated nucleic acid is detectably labeled, e.g., fluorescent, enzyme, radionuclide, or chemiluminescent label, and applied to a microarray, e.g., the specialized microarrays provided by this invention. The array is then washed to remove unbound material and visualized by staining or fluorescence, or other means known in the art depending on the type of label utilized.

In another embodiment of this invention, the predictor polynucleotides can be used as biomarkers for cells that are resistant or sensitive to c-met inhibitors. With the predictor polynucleotides in hand, screening and detection assays can be carried out to determine whether or not a given drug, preferably a c-met inhibitor, elicits a sensitive or a resistant phenotype following exposure of cells, e.g., cells taken from a tumor or cancer biopsy sample to the inhibitor. Thus, methods of screening, monitoring, detecting, prognosing and/or diagnosing to determine the resistance or sensitivity of cells to a drug that interacts with a c-met, or a c-met pathway, preferably a c-met inhibitor, and to which the cells are exposed, are encompassed by the present invention.

Suitable methods include detection and evaluation of polynucleotide activation or expression at the level of nucleic acid, e.g., DNA, RNA, mRNA, and detection and evaluation of encoded protein. For example, PCR assays as known and practiced in the art can be employed to quantify RNA or DNA in cells being assayed for susceptibility to drug treatment, for example, c-met kinase inhibitors.

In another embodiment, the invention is directed to a method of identifying cells, tissues, and/or patients that are predicted to be resistant to drugs that affect c-met signaling pathways, e.g., c-met, or that are resistant in different biological systems to those drugs. The method comprises the step(s) of (i) analyzing the expression of only those polynucleotides that have been shown to be correlative to predicting resistant responses to such compounds; (ii) comparing the observed expression levels of those correlative resistant polynucleotides in the test cells, tissues, and/or patients to the expression levels of those same polynucleotides in a cell line that is known to be resistant to the compounds; and (iii) predicting whether the cells, tissues, and/or patients are resistant to the compounds based upon the overall similarity of the observed expression of those polynucleotides in step (ii).

In another embodiment, the invention is directed to a method of identifying cells, tissues, and/or patients that are predicted to be sensitive to drugs that affect c-met signaling pathways, e.g., c-met, or that are sensitive in different biological systems to those drugs. The method involves the step(s) of (i) analyzing the expression of polynucleotides that have been shown to be correlative to predicting sensitive responses to such drugs; (ii) comparing the observed expression levels of those correlative sensitive polynucleotides in the test cells, tissues, and/or patients to the expression levels of those same polynucleotides in a cell line that is known to be sensitive to the drugs; and (iii) predicting whether the cells, tissues, and/or patients are sensitive to the drugs based upon the overall similarity of the observed expression of those polynucleotides in step (ii).

The invention further encompasses the detection and/or quantification of one or more of the c-met biomarker proteins of the present invention using antibody-based assays (e.g., immunoassays) and/or detection systems. Such assays include the following non-limiting examples, ELISA, immunofluorescence, fluorescence activated cell sorting (FACS), Western Blots, etc., as further described herein.

In another embodiment, the human c-met biomarker polypeptides and/or peptides of the present invention, or immunogenic fragments or oligopeptides thereof, can be used for screening therapeutic drugs in a variety of drug screening techniques. The fragment employed in such a screening assay can be free in solution, affixed to a solid support, borne on a cell surface, or located intracellularly. The reduction or abolition of activity of the formation of binding complexes between the biomarker protein and the agent being tested can be measured. Thus, the invention provides a method for screening or assessing a plurality of compounds for their specific binding affinity with an inhibitor biomarker polypeptide, or a bindable peptide fragment thereof, of this invention. The method comprises the steps of providing a plurality of compounds; combining the inhibitor biomarker polypeptide, or a bindable peptide fragment thereof, with each of the plurality of compounds, for a time sufficient to allow binding under suitable conditions; and detecting binding of the biomarker polypeptide or peptide to each of the plurality of test compounds, thereby identifying the compounds that specifically bind to the biomarker polypeptide or peptide. More specifically, the biomarker polypeptide or peptide is that of a c-met inhibitor biomarkers.

Methods to identify drugs that modulate the activity of the human c-met biomarker polypeptides by the present invention, comprise combining a candidate drug modulator of c-met and measuring an effect of the candidate drug modulator on the biological activity of the c-met inhibitor biomarker polypeptide or peptide. Such measurable effects include, for example, a physical binding interaction; the ability to cleave a suitable protein kinase substrate; effects on a native and cloned protein kinase biomarker-expressing cell line; and effects of modulators or other protein kinase-mediated physiological measures.

Another method of identifying drugs that modulate the biological activity of the c-met biomarker polypeptides of the present invention comprises combining a potential or candidate drug modulator of a c-met biological activity, e.g., c-met, with a host cell that expresses the c-met biomarker polypeptide and measuring an effect of the candidate drug modulator on the biological activity of the c-met biomarker polypeptides. The host cell can also be capable of being induced to express the c-met biomarker polypeptide, e.g., via inducible expression. Physiological effects of a given modulator candidate on the c-met biomarker polypeptide can also be measured. Thus, cellular assays for particular c-met modulators can be either direct measurement or quantification of the physical biological activity of the c-met biomarker polypeptide, or they may be measurement or quantification of a physiological effect. Such methods preferably employ a c-met biomarker polypeptide as described herein, or an overexpressed recombinant c-met biomarker polypeptide in suitable host cells containing an expression vector as described herein, wherein the c-met biomarker polypeptide is expressed, overexpressed, or undergoes up-regulated expression.

Another aspect of the present invention embraces a method of screening for a drug that is capable of modulating the biological activity of a c-met biomarker polypeptide. The method comprises providing a host cell containing an expression vector harboring a nucleic acid sequence encoding a c-met biomarker polypeptide, or a functional peptide or portion thereof; determining the biological activity of the expressed c-met biomarker polypeptide in the absence of a modulator compound; contacting the cell with the modulator compound and determining the biological activity of the expressed c-met biomarker polypeptide in the presence of the modulator compound. In such a method, a difference between the activity of the c-met biomarker polypeptide in the presence of the modulator compound and in the absence of the modulator compound indicates a modulating effect of the compound.

The term "drug" is used herein to refer to a substance that potentially can be used as a medication or in the preparation of a medication. Essentially any chemical compound can be employed as a drug in the assays according to the present invention. Compounds tested can be any small chemical compound, or biological entity (e.g., amino acid chain, protein, sugar, nucleic acid, or lipid). Test compounds are typically small chemical molecules and peptides. Generally, the compounds used as potential modulators can be dissolved in aqueous or organic (e.g., DMSO-based) solutions. The assays are designed to screen large chemical libraries by automating the assay steps and providing compounds from any convenient source. Assays are typically run in parallel, for example, in microtiter formats on microtiter plates in robotic assays. There are many suppliers of chemical compounds, including, for example, Sigma (St. Louis, Mo.), Aldrich (St. Louis, Mo.), Sigma-Aldrich (St. Louis, Mo.), Fluka Chemika-Biochemica Analytika (Buchs, Switzerland). Also, compounds can be synthesized by methods known in the art.

High throughput screening methodologies are particularly envisioned for the detection of modulators of the novel c-met biomarker, polynucleotides and polypeptides described herein. Such high throughput screening methods typically involve providing a combinatorial chemical or peptide library containing a large number of potential therapeutic compounds (e.g., ligand or modulator compounds). The combinatorial chemical libraries or ligand libraries are then screened in one or more assays to identify those library members (e.g., particular chemical species or subclasses) that display a desired characteristic activity. The compounds so identified can serve as conventional lead compounds, or can themselves be used as potential or actual therapeutics.

In another of its aspects, the invention encompasses screening and small molecule (e.g., drug) detection assays which involve the detection or identification of small molecules that can bind to c-met biomarker polypeptide or peptide. Particularly preferred are assays suitable for high throughput screening methodologies.

In such binding-based detection, identification, or screening assays, a functional assay is not typically required. All that is needed, in general, is a target protein, preferably substantially purified, and a library or panel of compounds (e.g., ligands, drugs, or small molecules), or biological entities to be screened or assayed for binding to the protein target. Preferably, most small molecules that bind to the target protein modulate the target's activity in some manner due to preferential, higher affinity binding to functional areas or sites on the protein.

An example of such an assay is the fluorescence based thermal shift assay described in U.S. Patents Nos. 6,020,141 and 6,036,920 to Pantoliano et al. (See also, J. Zimmerman, 2000, Gen. Eng. News, 20(8)). The assay allows the detection of small molecules that bind to expressed, and preferably purified, c-met, based on affinity of binding determinations by analyzing thermal unfolding curves of protein-drug or ligand complexes. The drugs or binding molecules determined by this technique can be further assayed, if desired, by methods such as those described herein to determine if the molecules affect or modulate function or activity of the target protein.

To purify c-met biomarker polypeptide, to measure a biological binding or ligand binding activity, the source may be a whole cell lysate that can be prepared by successive freeze-thaw cycles (e.g., one to three) in the presence of standard protease inhibitors. The c-met biomarker polypeptide can be partially or completely purified by standard protein purification methods, e.g., affinity chromatography using specific antibody(ies) described herein, or by ligands specific for an epitope tag engineered into the recombinant c-met biomarker polypeptide molecule, also as described herein. Binding activity can then be measured as described.

Drugs which are identified according to the methods provided herein, and which modulate or regulate the biological activity or physiology of the c-met biomarker polypeptides according to the present invention, are a preferred embodiment of this invention. It is contemplated that such modulatory compounds can be employed in treatment and therapeutic methods for treating a condition that is mediated by the c-met biomarker polypeptides by administering to an individual in need of such treatment a therapeutically effective amount of the compound identified by the methods described herein.

In addition, the present invention provides methods for treating an individual in need of such treatment for a disease, disorder, or condition that is mediated by the c-met biomarker polypeptides of the invention, comprising administering to the individual a therapeutically effective amount of the c-met biomarker-modulating compound identified by a method provided herein.

The present invention particularly provides methods for treating an individual in need of such treatment for a disease, disorder, or condition that is mediated by c-met biomarker polypeptides of the invention, comprising administering to the individual a therapeutically effective amount of the c-met biomarker-modulating compound identified by a method provided herein.

The present invention further encompasses polypeptides comprising, or alternatively consisting of, an epitope of the polypeptide having an amino acid sequence of one or more of the c-met biomarkers. The present invention also encompasses polynucleotide sequences encoding an epitope of a polypeptide sequence of the c-met biomarkers of the invention.

The term "epitopes" as used herein, refers to portions of a polypeptide having antigenic or immunogenic activity in an animal, preferably a mammal, and most preferably a human. In a preferred embodiment, the present invention encompasses a polypeptide comprising an epitope, as well as the polynucleotide encoding this polypeptide. An "immunogenic epitope" as used herein, refers to a portion of a protein that elicits an antibody response in an animal, as determined by any method known in the art, for example, by the methods for generating antibodies described infra. (See, for example, Geysen et al., 1983, Proc. Natl. Acad. Sci. USA, 81:3998-4002). The term "antigenic epitope" as used herein refers to a portion of a protein to which an antibody can immunospecifically bind to its antigen as determined by any method well known in the art, for example, by the immunoassays described herein. Immunospecific binding excludes non-specific binding, but does not necessarily exclude cross-reactivity with other antigens. Antigenic epitopes need not necessarily be immunogenic. Either the full-length protein or an antigenic peptide fragment can be used. Antibodies are preferably prepared from these regions or from discrete fragments in regions of the tyrosine kinase biomarker nucleic acid and protein sequences comprising an epitope. Polypeptide or peptide fragments that function as epitopes may be produced by any conventional means. (See, e.g., Houghten, 1985, Proc. Natl. Acad. Sci. USA, 82:5131-5135; and as described in U.S. Pat. No. 4,631,211).

Moreover, antibodies can also be prepared from any region of the polypeptides and peptides of the c-met kinase biomarkers as described herein. In addition, if a polypeptide is a receptor protein, antibodies can be developed against an entire receptor or portions of the receptor, for example, the intracellular carboxy terminal domain, the amino terminal extracellular domain, the entire transmembrane domain, specific transmembrane segments, any of the intracellular or extracellular loops, or any portions of these regions. Antibodies can also be developed against specific functional sites, such as the site of ligand binding, or sites that are glycosylated, phosphorylated, myristylated, or amidated, for example.

In the present invention, antigenic epitopes for generating antibodies preferably contain a sequence of at least 4, at least 5, at least 6, at least 7, more preferably at least 8, at least 9, at least 10, at least 11, at least 12, at least 13, at least 14, at least 15, at least 20, at least 25, at least 30, at least 40, at least 50, and, most preferably, between about 15 to about 30 amino acid residues. Combinations of the foregoing epitopes are included. Preferred polypeptides comprising immunogenic or antigenic epitopes are at least 10, 15, 20, 25, 30, 35, 40, 45, 50, 55, 60, 65, 70, 75, 80, 85, 90, 95, or 100 amino acid residues in length. Additional non-exclusive preferred antigenic epitopes include the antigenic epitopes disclosed herein, as well as portions thereof, as well as any combination of two, three, four, five or more of these antigenic epitopes. Such antigenic epitopes can be used as the target molecules in immunoassays. (See, for instance, Wilson et al., 1984, Cell, 37:767-778; and Sutcliffe et al., 1983, Science, 219:660-666). The fragments as described herein are not to be construed, however, as encompassing any fragments which may be disclosed prior to the invention.

C-met biomarker polypeptides comprising one or more immunogenic epitopes which elicit an antibody response can be introduced together with a carrier protein, such as an albumin, to an animal system (such as rabbit or mouse). Alternatively, if the polypeptide is of sufficient length (e.g., at least about 15-25 amino acids), the polypeptide can be presented without a carrier. However, immunogenic epitopes comprising as few as 5 to 10 amino acids have been shown to be sufficient to raise antibodies capable of binding to, at the very least, linear epitopes in a denatured polypeptide (e.g., in Western blotting).

Epitope-bearing polypeptides of the present invention can be used to induce antibodies according to methods well known in the art including, but not limited to, in vivo immunization, in vitro immunization, and phage display methods. See, e.g., Sutcliffe et al., supra; Wilson et al., supra; and Bittle et al., supra). If in vivo immunization is used, animals can be immunized with free peptide of appropriate size; however, the anti-peptide antibody titer can be boosted by coupling the peptide to a macromolecular carrier, such as keyhole limpet hemacyanin (KLH), or tetanus toxoid (TT). For instance, peptides containing cysteine residues can be coupled to a carrier using a linker such as maleimidobenzoyl-N-hydroxysuccinimide ester (MBS), while other peptides may be coupled to carriers using a more general linking agent, such as glutaraldehyde.

Peptides containing epitopes can also be synthesized as multiple antigen peptides (MAPs), first described by J. P. Tam et al. (1995, Biomed. Pept., Proteins, Nucleic Acids, 199, 1(3):123-32) and Calvo et al. (1993, J. Immunol., 150(4):1403-12), which are hereby incorporated by reference in their entirety herein. MAPs contain multiple copies of a specific peptide attached to a non-immunogenic lysine core. MAP peptides usually contain four or eight copies of the peptide, which are often referred to as MAP4 or MAP8 peptides. By way of non-limiting example, MAPs can be synthesized onto a lysine core matrix attached to a polyethylene glycol-polystyrene (PEG-PS) support. The peptide of interest is synthesized onto the lysine residues using 9-fluorenylmethoxycarbonyl (Fmoc) chemistry. For example, Applied Biosystems (Foster City, Calif.) offers commercially available MAP resins, such as, for example, the Fmoc Resin 4 Branch and the Fmoc Resin 8 Branch which can be used to synthesize MAPs. Cleavage of MAPs from the resin is performed with standard trifloroacetic acid (TFA)-based cocktails known in the art. Purification of MAPs, except for desalting, is not generally necessary. MAP peptides can be used in immunizing vaccines which elicit antibodies that recognize both the MAP and the native protein from which the peptide was derived.

Epitope-bearing peptides of the invention can also be incorporated into a coat protein of a virus, which can then be used as an immunogen or a vaccine with which to immunize animals, including humans, in order stimulate the production of anti-epitope antibodies. For example, the V3 loop of the gp120 glycoprotein of the human immunodeficiency virus type 1 (HIV-1) has been engineered to be expressed on the surface of rhinovirus. Immunization with rhinovirus displaying the V3 loop peptide yielded apparently effective mimics of the HIV-1 immunogens (as measured by their ability to be neutralized by anti-HIV-1 antibodies as well as by their ability to elicit the production of antibodies capable of neutralizing HIV-1 in cell culture). This techniques of using engineered viral particles as immunogens is described in more detail in Smith et al., 1997, Behring Inst Mitt Feb, (98):229-39; Smith et al., 1998, J. Virol., 72:651-659; and Zhang et al., 1999, Biol. Chem., 380:365-74), which are hereby incorporated by reference herein in their entireties.

Moreover, polypeptides or peptides containing epitopes according to the present invention can be modified, for example, by the addition of amino acids at the amino-and/or carboxy-terminus of the peptide. Such modifications are performed, for example, to alter the conformation of the epitope bearing polypeptide such that the epitope will have a conformation more closely related to the structure of the epitope in the native protein. An example of a modified epitope-bearing polypeptide of the invention is a polypeptide in which one or more cysteine residues have been added to the polypeptide to allow for the formation of a disulfide bond between two cysteines, thus resulting in a stable loop structure of the epitope-bearing polypeptide under non-reducing conditions. Disulfide bonds can form between a cysteine residue added to the polypeptide and a cysteine residue of the naturally-occurring epitope, or between two cysteines which have both been added to the naturally-occurring epitope-bearing polypeptide.

In addition, it is possible to modify one or more amino acid residues of the naturally-occurring epitope-bearing polypeptide by substitution with cysteines to promote the formation of disulfide bonded loop structures. Cyclic thioether molecules of synthetic peptides can be routinely generated using techniques known in the art, e.g., as described in PCT publication WO 97/46251, incorporated in its entirety by reference herein. Other modifications of epitope-bearing polypeptides contemplated by this invention include biotinylation.

For the production of antibodies in vivo, host animals, such as rabbits, rats, mice, sheep, or goats, are immunized with either free or carrier-coupled peptides or MAP peptides, for example, by intraperitoneal and/or intradermal injection. Injection material is typically an emulsion containing about 100 .mu.g of peptide or carrier protein and Freund's adjuvant, or any other adjuvant known for stimulating an immune response. Several booster injections may be needed, for instance, at intervals of about two weeks, to provide a useful titer of anti-peptide antibody which can be detected, for example, by ELISA assay using free peptide adsorbed to a solid surface. The titer of anti-peptide antibodies in serum from an immunized animal can be increased by selection of anti-peptide antibodies, e.g., by adsorption of the peptide onto a solid support and elution of the selected antibodies according to methods well known in the art.

The polypeptides of the present invention can be fused with the constant domain of immunoglobulins (IgA, IgE, IgG, IgD, or IgM), or portions thereof, e.g., CH1, CH2, CH3, or any combination thereof, and portions thereof, or with albumin (including, but not limited to, recombinant human albumin, or fragments or variants thereof (see, e.g., U.S. Pat. No. 5,876,969; EP Patent No. 0 413 622; and U.S. Pat. No. 5,766,883, incorporated by reference in their entirety herein), thereby resulting in chimeric polypeptides. Such fusion proteins may facilitate purification and may increase half-life in vivo. This has been shown for chimeric proteins containing the first two domains of the human CD4-polypeptide and various domains of the constant regions of the heavy or light chains of mammalian immunoglobulins. (see, e.g., Traunecker et al., 1988, Nature, 331:84-86).

Enhanced delivery of an antigen across the epithelial barrier to the immune system has been demonstrated for antigens (e.g., insulin) conjugated to an FcRn binding partner, such as IgG or Fc fragments (see, e.g., WO 96/22024 and WO 99/04813). IgG fusion proteins that have a disulfide-linked dimeric structure due to the IgG portion disulfide bonds have also been found to be more efficient in binding and neutralizing other molecules than are monomeric polypeptides, or fragments thereof, alone. (See, e.g., Fountoulakis et al., 1995, J. Biochem., 270:3958-3964).

Nucleic acids encoding epitopes can also be recombined with a polynucleotide of interest as an epitope tag (e.g., the hemagglutinin ("HA") tag or flag tag) to aid in detection and purification of the expressed polypeptide. For example, a system for the ready purification of non-denatured fusion proteins expressed in human cell lines has been described by Janknecht et al., (1991, Proc. Natl. Acad. Sci. USA, 88:8972-897). In this system, the polynucleotide of interest is subcloned into a vaccinia recombination plasmid such that the open reading frame of the polynucleotide is translationally fused to an amino-terminal tag having six histidine residues. The tag serves as a matrix binding domain for the fusion protein. Extracts from cells infected with the recombinant vaccinia virus are loaded onto an Ni.sup.2+ nitriloacetic acid-agarose column and histidine-tagged proteins are selectively eluted with imidazole-containing buffers.

Additional fusion proteins of the invention can be generated by employing the techniques of gene-shuffling, motif-shuffling, exon-shuffling, and/or codon-shuffling (collectively referred to as "DNA shuffling"). DNA shuffling can be employed to modulate the activities of polypeptides of the invention; such methods can be used to generate polypeptides with altered activity, as well as agonists and antagonists of the polypeptides. See, generally, U.S. Pat. Nos. 5,605,793; 5,811,238; 5,830,721; 5,834,252; and 5,837,458, and Patten et al., 1997, Curr. Opinion Biotechnol., 8:724-33; Harayama, 1998, Trends Biotechnol., 16(2):76-82; Hansson, et al., 1999, J. Mol. Biol., 287:265-76; and Lorenzo and Blasco, 1998, Biotechniques, 24(2):308-313, the contents of each of which are hereby incorporated by reference in its entirety.

In an embodiment of the invention, alteration of polynucleotides corresponding to one or more of the c-met biomarker polynucleotide sequences and the polypeptides encoded by these polynucleotides, can be achieved by DNA shuffling. DNA shuffling involves the assembly of two or more DNA segments by homologous or site-specific recombination to generate variation in the polynucleotide sequence. In another embodiment, polynucleotides of the invention, or their encoded polypeptides, may be altered by being subjected to random mutapolynucleotidesis by error-prone PCR, random nucleotide insertion, or other methods, prior to recombination. In another embodiment, one or more components, motifs, sections, parts, domains, fragments, etc., of a polynucleotide encoding a polypeptide of this invention may be recombined with one or more components, motifs, sections, parts, domains, fragments, etc. of one or more heterologous molecules.

A bispecific or bifunctional antibody is an artificial hybrid antibody having two different heavy/light chain pairs and two different binding sites. Bispecific antibodies can be produced by a variety of methods, including fusion of hybridomas or linking of Fab' fragments. (See, e.g., Songsivilai & Lachmann, 1990, Clin. Exp. Immunol., 79:315-321; Kostelny et al., 1992, J. Immunol., 148:1547 1553). In addition, bispecific antibodies can be formed as "diabodies" (See, Holliger et al., 1993, Proc. Natl. Acad. Sci. USA, 90:6444-6448), or "Janusins" (See, Traunecker et al., 1991, EMBO J., 10:3655-3659 and Traunecker et al., 1992, Int. J. Cancer Suppl. 7:51-52-127).

Antibodies of the invention include, but are not limited to, polyclonal, monoclonal, multispecific, human, humanized or chimeric antibodies, single chain antibodies, Fab fragments, F(ab') fragments, fragments produced by a Fab expression library, anti-idiotypic (anti-Id) antibodies (including, e.g., anti-Id antibodies to antibodies of the invention), intracellularly made antibodies (i.e., intrabodies), and epitope-binding fragments of any of the above. The term "antibody", as used herein, refers to immunoglobulin molecules and immunologically active portions or fragments of immunoglobulin molecules, i.e., molecules that contain an antigen binding site that immunospecifically binds an antigen. The immunoglobulin molecules of the invention can be of any type (e.g., IgG, IgE, IgM, IgD, IgA and IgY), class or subclass (e.g., IgG1, IgG2, IgG3, IgG4, IgA1 and IgA2) of immunoglobulin molecule. Preferably, immunoglobulin is an IgG1, an IgG2, or an IgG4 isotype.

Immunoglobulins may have both a heavy and a light chain. An array of IgG, IgE, IgM, IgD, IgA, and IgY heavy chains can be paired with a light chain of the kappa or lambda types. Most preferably, the antibodies of the present invention are human antigen-binding antibodies and antibody fragments and include, but are not limited to, Fab, Fab' F(ab') 2, Fd, single-chain Fvs (scFv), single-chain antibodies, disulfide-linked Fvs (sdFv) and fragments comprising either a V.sub.L or V.sub.H domain. Antigen-binding antibody fragments, including single-chain antibodies, can comprise the variable region(s) alone or in combination with the entirety or a portion of the following: hinge region, and CH1, CH2, and CH3 domains. Also included in connection with the invention are antigen-binding fragments comprising any combination of variable region(s) with a hinge region, and CH1, CH2, and CH3 domains. The antibodies of the invention can be from any animal origin including birds and mammals. Preferably, the antibodies are of human, murine (e.g., mouse and rat), donkey, sheep, rabbit, goat, guinea pig, camel, horse, or chicken origin. As used herein, "human" antibodies include antibodies having the amino acid sequence of a human immunoglobulin and include antibodies isolated from human immunoglobulin libraries or from animals transgenic for one or more human immunoglobulin and that do not express endogenous immunoglobulins, as described infra and, for example, in U.S. Pat. No. 5,939,598.

The antibodies of the present invention can be monospecific, bispecific, trispecific, or of greater multispecificity. Multispecific antibodies can be specific for different epitopes of a polypeptide of the present invention, or can be specific for both a polypeptide of the present invention, and a heterologous epitope, such as a heterologous polypeptide or solid support material. (See, e.g., WO 93/17715; WO 92/08802; WO 91/00360; WO 92/05793; Tutt et al., 1991, J. Immunol., 147:60-69; U.S. Pat. Nos. 4,474,893; 4,714,681; 4,925,648; 5,573,920; 5,601,819; and Kostelny et al., 1992, J. Immunol., 148:1547-1553).

Antibodies of the present invention can be described or specified in terms of the epitope(s) or portion(s) of a polypeptide of the present invention which they recognize or specifically bind. The epitope(s) or polypeptide portion(s) can be specified, e.g., by N-terminal and C-terminal positions, by size in contiguous amino acid residues, or as presented in the sequences defined in Table 2 herein. Further included in accordance with the present invention are antibodies which bind to polypeptides encoded by polynucleotides which hybridize to a polynucleotide of the present invention under stringent, or moderately stringent, hybridization conditions as described herein.

The antibodies of the invention (including molecules comprising, or alternatively consisting of, antibody fragments or variants thereof) can bind immunospecifically to a polypeptide or polypeptide fragment or to a variant human c-met kinase biomarker of the invention, e.g., the Src biomarker proteins as set forth in Table 2, and/or monkey src biomarker protein.

By way of non-limiting example, an antibody can be considered to bind to a first antigen preferentially if it binds to the first antigen with a dissociation constant (Kd) that is less than the antibody's Kd for the second antigen. In another non-limiting embodiment, an antibody can be considered to bind to a first antigen preferentially if it binds to the first antigen with an affinity that is at least one order of magnitude less than the antibody's Ka for the second antigen. In another non-limiting embodiment, an antibody can be considered to bind to a first antigen preferentially if it binds to the first antigen with an affinity that is at least two orders of magnitude less than the antibody's Kd for the second antigen.

In another nonlimiting embodiment, an antibody may be considered to bind to a first antigen preferentially if it binds to the first antigen with an off rate (koff) that is less than the antibody's koff for the second antigen. In another nonlimiting embodiment, an antibody can be considered to bind to a first antigen preferentially if it binds to the first antigen with an affinity that is at least one order of magnitude less than the antibody's koff for the second antigen. In another nonlimiting embodiment, an antibody can be considered to bind to a first antigen preferentially if it binds to the first antigen with an affinity that is at least two orders of magnitude less than the antibody's koff for the second antigen.

The present invention also embraces a kit for determining, predicting, or prognosing drug susceptibility or resistance by a patient having a disease, particularly a cancer or tumor, preferably, a breast cancer or tumor. Such kits are useful in a clinical setting for use in testing patient's biopsied tumor or cancer samples, for example, to determine or predict if the patient's tumor or cancer will be resistant or sensitive to a given treatment or therapy with a drug, compound, chemotherapy agent, or biological treatment agent. Provided in the kit are the predictor set comprising those polynucleotides correlating with resistance and sensitivity to c-met kinase modulators in a particular biological system, particularly c-met kinase inhibitors, and preferably comprising a microarray; and, in suitable containers, the modulator compounds for use in testing cells from patient tissue or patient samples for resistance/sensitivity; and instructions for use.

Also, as explained above, the kit can encompass a variety of methods and systems by which the expression of the predictor/marker polynucleotides can be assayed and/or monitored, both at the level of mRNA and of protein, for example, via PCR assays, e.g., RT-PCR and immunoassay, such as ELISA. In kits for performing PCR, or in situ hybridization, for example, nucleic acid primers or probes from the sequences of one or more of the predictor polynucleotides, such as those described herein, in addition to buffers and reagents as necessary for performing the method, and instructions for use. In kits for performing immunoassays, e.g. ELISAs, immunoblotting assays, and the like, antibodies, or bindable portions thereof, to the c-met kinase biomarker polypeptides of the invention, or to antigenic or immunogenic peptides thereof, are supplied, in addition to buffers and reagents as necessary for performing the method, and instructions for use.

In another embodiment, the present invention embraces the use of one or more polynucleotides among those of the predictor polynucleotides identified herein that can serve as targets for the development of drug therapies for disease treatment. Such targets may be particularly applicable to treatment of breast diseases, such as breast cancers or tumors. Indeed, because these predictor polynucleotides are differently expressed in sensitive and resistant cells, their expression pattern is correlated with relative intrinsic sensitivity of cells to treatment with compounds that interact with and inhibit c-met kinases. Accordingly, the polynucleotides highly expressed in resistant cells can serve as targets for the development of drug therapies for the tumors which are resistant to c-met kinase inhibitor compounds, for example, c-met kinase inhibitors.

### EXAMPLES

The Examples herein are meant to exemplify the various aspects of carrying out the invention and are not intended to limit the scope of the invention in any way. The Examples do not include detailed descriptions for conventional methods employed, such as in the construction of vectors, the insertion of cDNA into such vectors, or the introduction of the resulting vectors into the appropriate host. Such methods are well known to those skilled in the art and are described in numerous publications, for example, Sambrook, Fritsch, and Maniatis, Molecular Cloning: A Laboratory Manual, 2nd Edition, Cold Spring Harbor Laboratory Press, USA, (1989).

### C-met biomarker studies

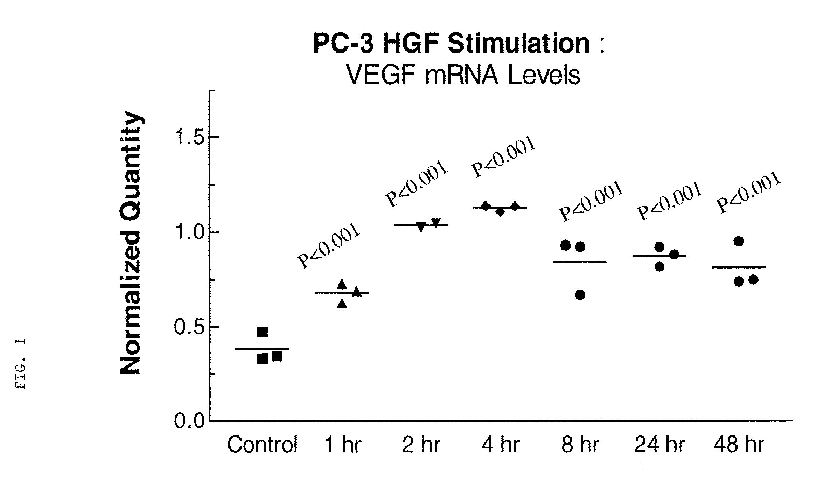

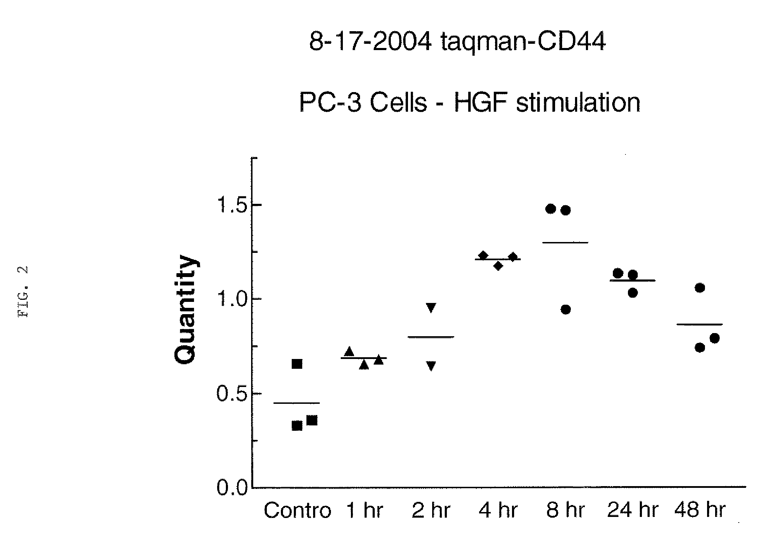

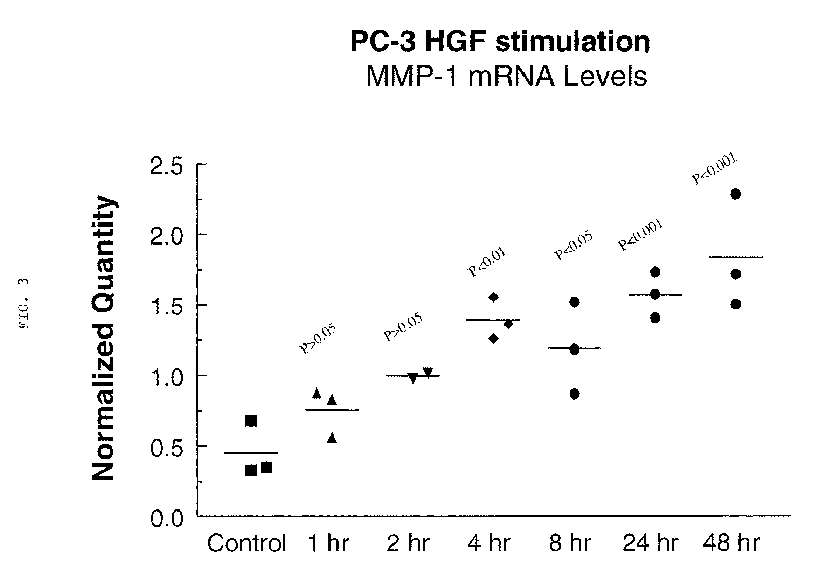

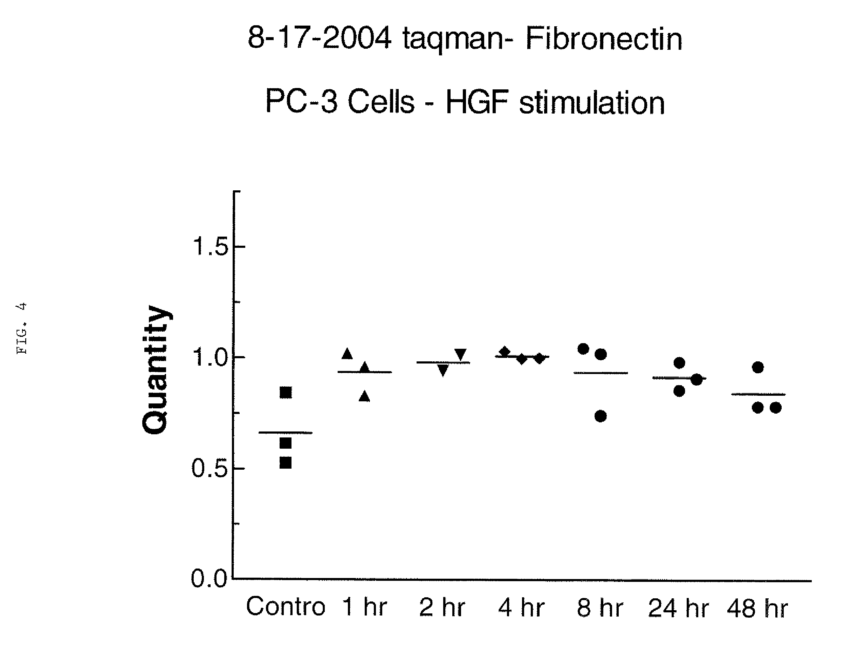

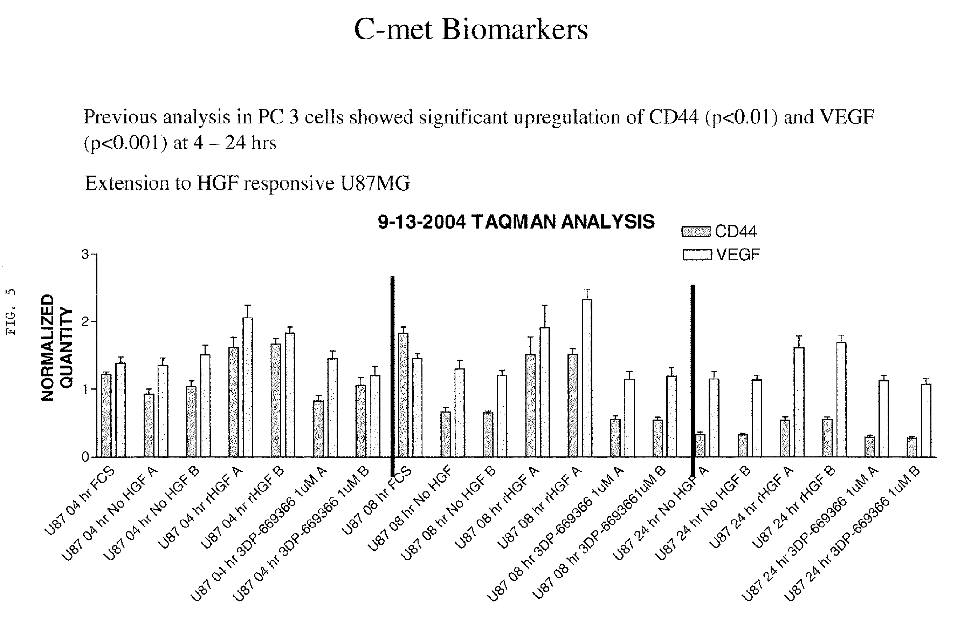

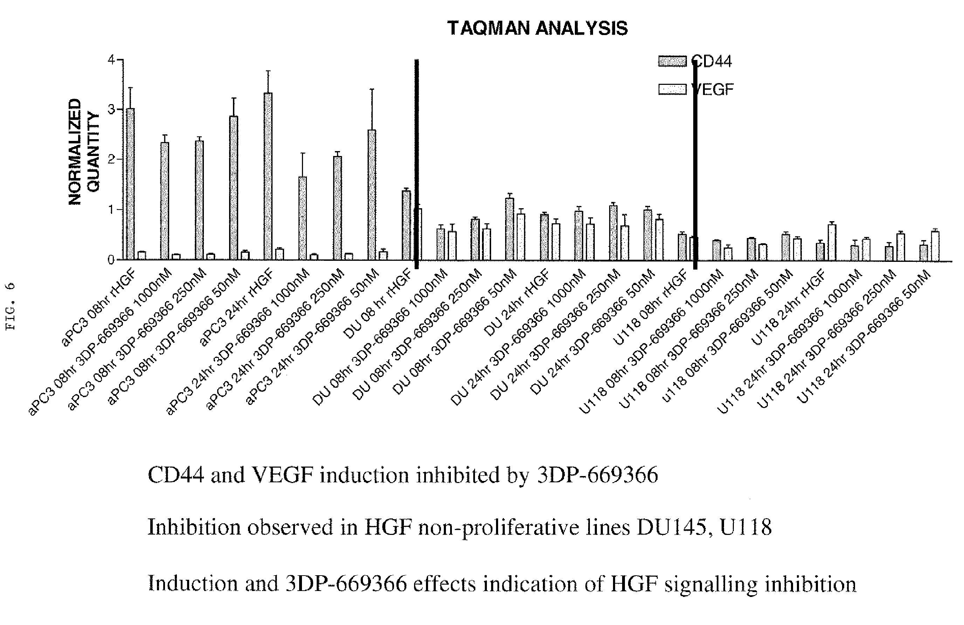

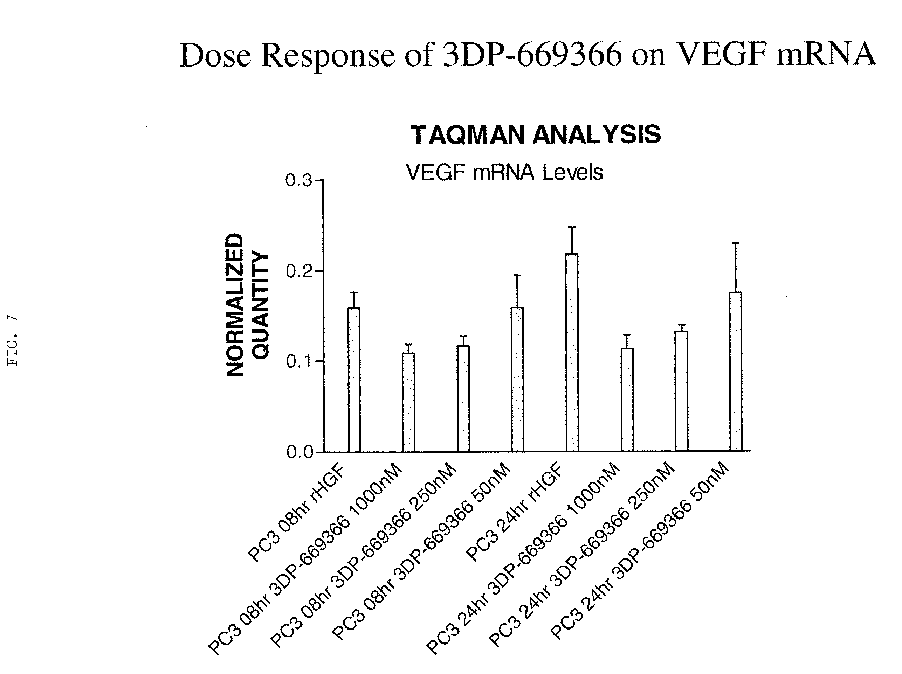

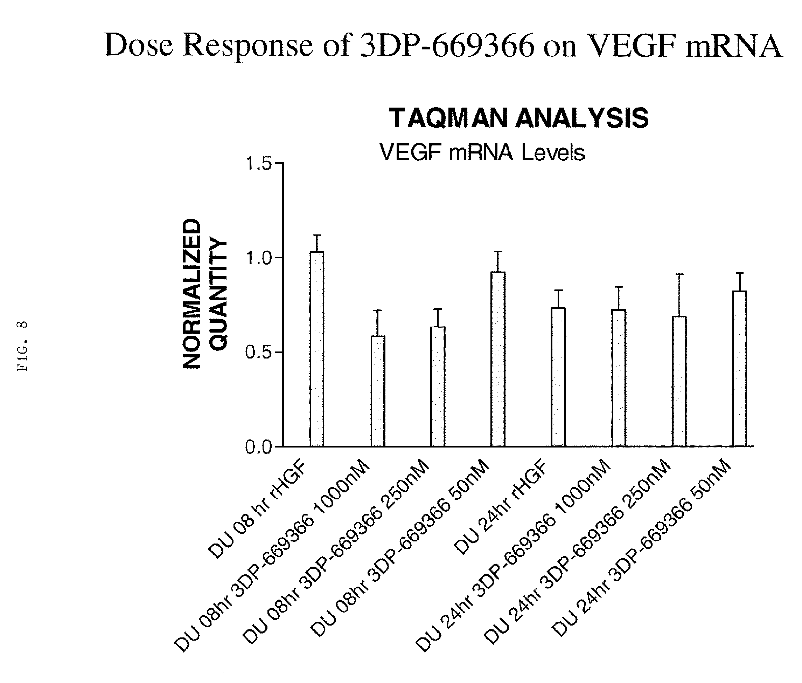

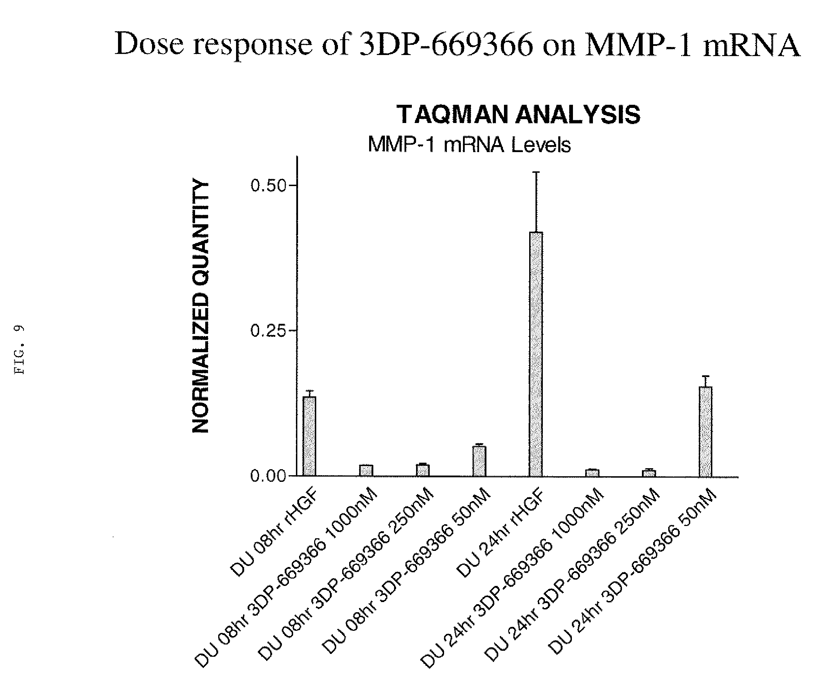

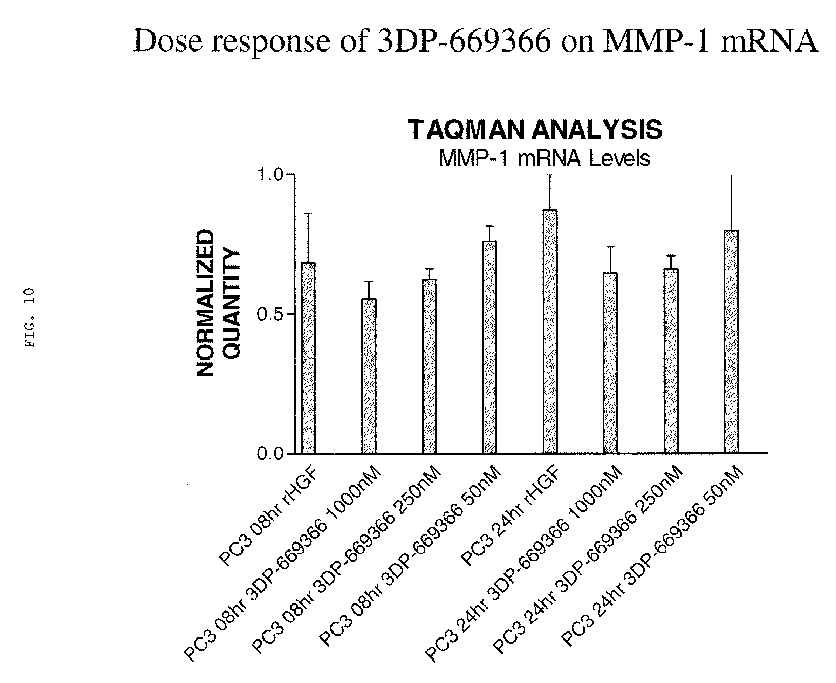

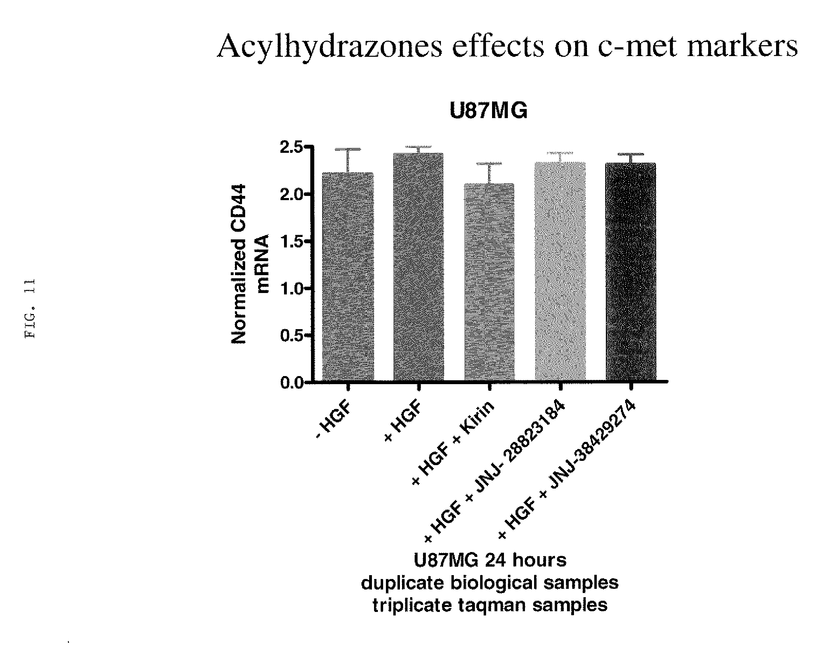

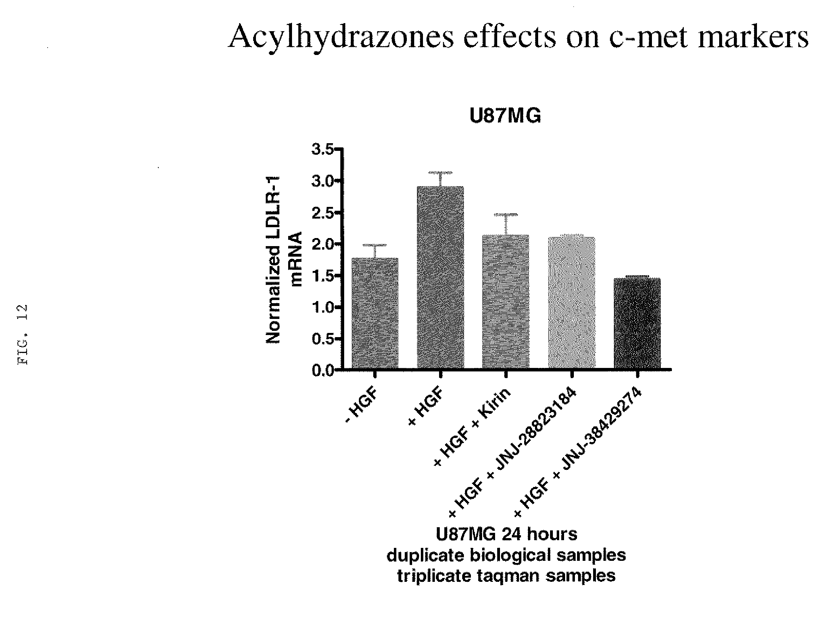

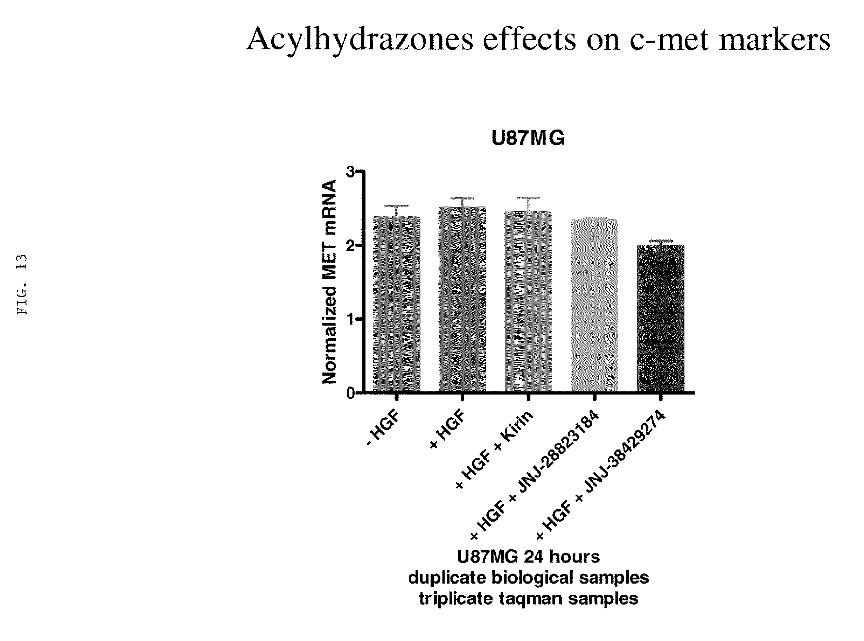

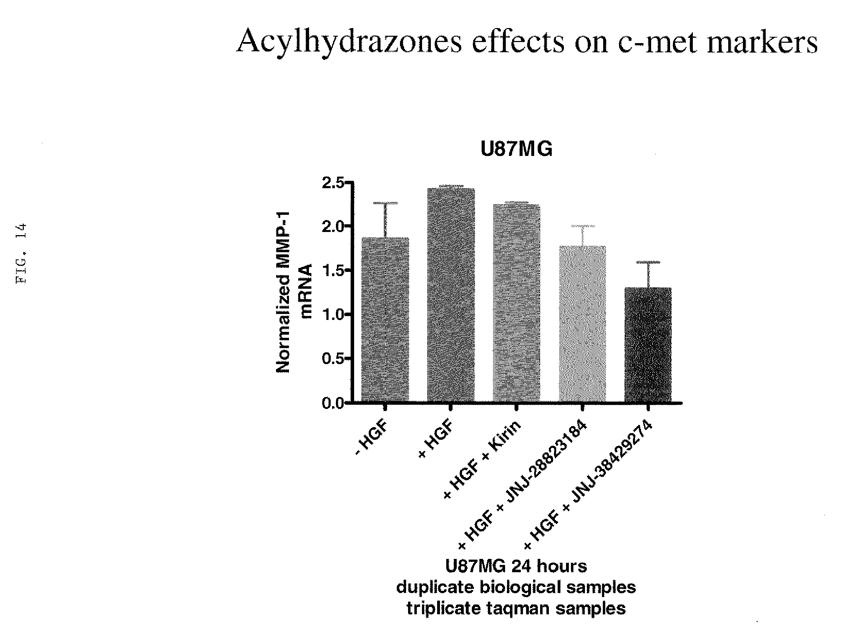

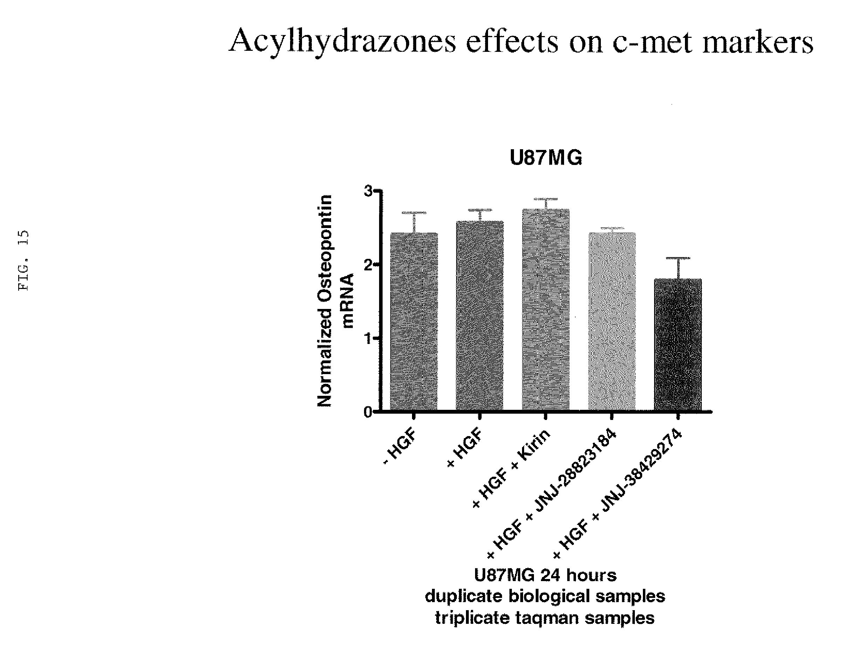

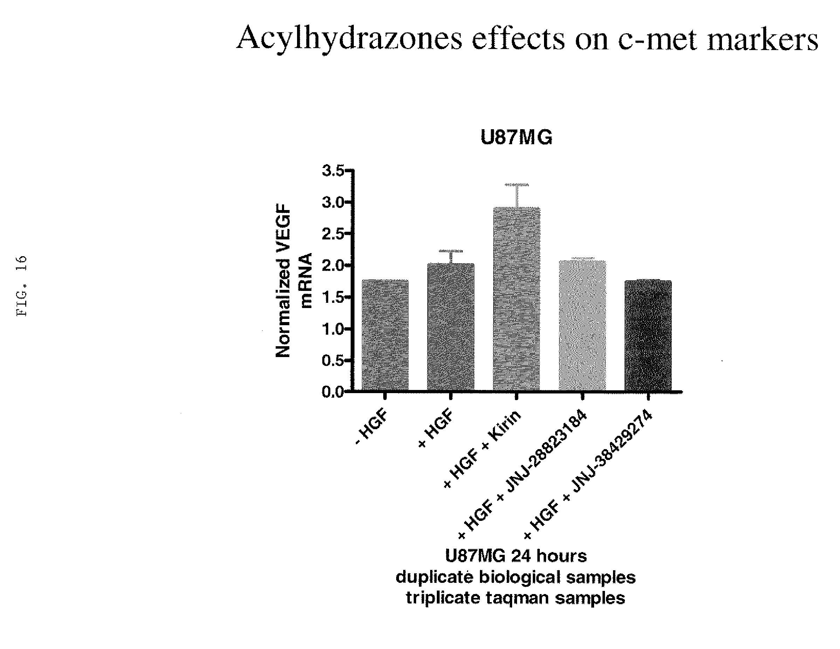

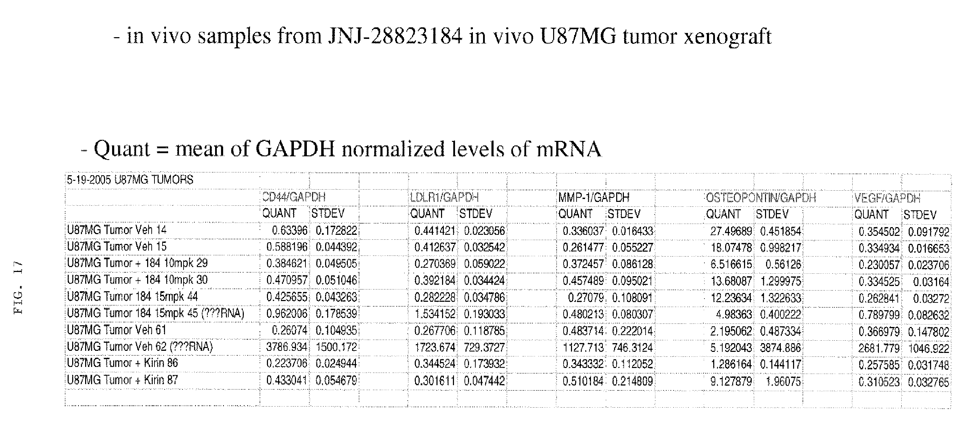

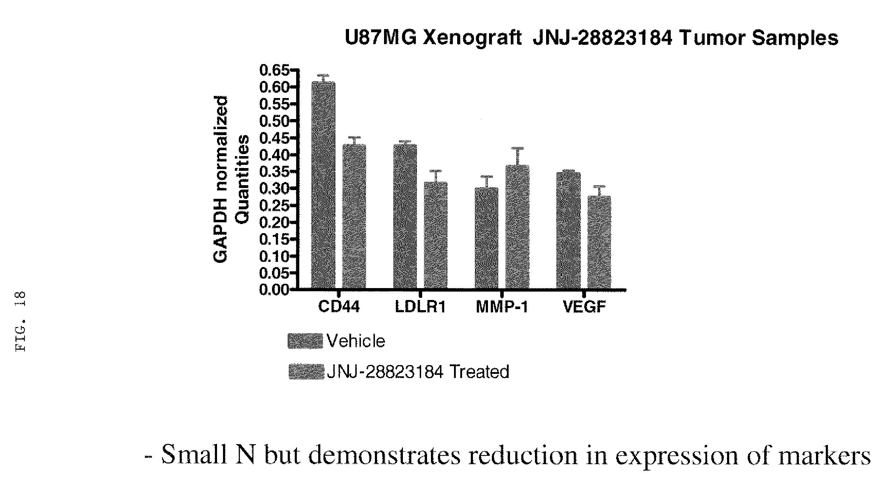

PC-3 prostate carcinoma cells and the U87MG glioblastoma line have been shown to proliferate in response to HGF stimulation. In order to identify biomarkers for the HGF response and potential inhibition of this response by small molecule HGF receptor (c-met) inhibitors, a micro array analysis of PC-3 cells stimulated by HGF over a time course of 1 hr to 2 days was used. TAQMAN was employed to examine mRNA levels of VEGF, CD44, Fibronectin, and MMP-1, each of which has been shown by others to be induced by HGF. Briefly, RNA is isolated by lysis of cultured cells or homogenization of tumor tissue in a guanidine solution, Trireagent from Molecular Research Center, Inc. The RNA is purified by a Qiagen column methodology under conditions recommended by the manufacturer and DNAse treated. Specific levels of mRNAs for the indicated genes are determined by Real-Time PCR, Taqman analysis using probes and primers specific for the genes examined and purchased from Applied Biosystems and the SDS2.1 quantitation software. Quantitation is performed by comparison of the cycle number where the sample crosses a threshold value vs a standard curve made of a pool of the RNA samples. Normalization is performed by comparing the amount of specific mRNA to that of 18S RNA or GAPDH mRNA. A statistically significant induction of VEGF (p<0.001), CD44 (p<0.01) and MMP-1 (p<0.01) in PC-3 cells at time points between 8 and 48 hours following HGF addition has been observed. See FIGS. 1-3, respectively. Fibronectin did not show a statistically significant induction in PC-3. See FIG. 4. This analysis was extended to U87MG glioblastoma cells, wherein similar induction of VEGF, CD44 and MMP-1 was observed. See FIG. 5. This response was then evaluated upon administration of the c-met inhibitor, 3DP-669366. Treatment of U87MG cells with 1uM 3DP-669366 resulted in the abrogation of the HGF stimulated induction of VEGF, CD44 and MMP-1 to non-stimulated levels at 8 and 24 hours. In PC-3 cells, a concentration dependent drop in mRNA levels of VEGF, CD44 and MMP-1 was observed at 8 and 24 hours. See FIG. 6. Similar drops in expression of VEGF, CD44 and MMP-1 was observed in DU145 and U118 prostate and glioblastoma cell lines. These lines do not proliferate in response to HGF but do show morphological changes upon HGF treatment. These results suggest that VEGF, CD44 and MMP-1 are markers for c-met signaling which reflect the inhibition of the receptor. See FIGS. 7-10. The kinetics of the induction and the amplitude of the mRNA levels differ between the cell lines examined. The micro-array analysis identified LDL Receptor 1 (LDL-R1) as another protein that is induced by HGF and whose level of expression is diminished by treatment of cells with a HGF receptor inhibitor. This analysis was then evaluated upon administration of the acylhydrazone series of c-met inhibitors, JNJ-28823184 and JNJ-38429274. See FIGS. 11-18. In in vivo tumor xenografts, the biomarkers identified by examination of cell culture responses to c-met inhibitors are also modulated in the in vivo setting. In a U87MG tumor xenograft in nude mice, a decrease in the mRNA levels of CD44, VEGF, LDLR1, and Osteopontin was observed.

These biomarkers may be used in accordance with the invention to assess response to c-met treatments in patients. For example, cells can be obtained, marker mRNA levels can be assessed by Taqman technology and inhibition or lack of inhibition of c-met can be determined in order to predict a clinical response.

### Aspects of the Invention

1. A biomarker whose expression pattern is predictive of a response of cells to treatment with a drug that inhibits c-met activity.
2. The biomarker according to aspect 1 selected from the group consisting of VEGF, CD44, MMP-1, osteopontin and LDL-R1.
3. A method for predicting whether a drug is capable of inhibiting c-met activity in a cell, comprising the steps of: a) obtaining a sample of cells; b) determining an expression pattern of said sample of cells; c) comparing said expression pattern with a predictor expression pattern, said predictor expression pattern including a biomarker of aspect 1; and d) correlating the expression pattern of said sample of cells to said drugs' ability or inability to inhibit c-met activity in said cells.
4. The method according to aspect 1 wherein the biomarker is a polynucleotide.
5. The method according to aspect 1 wherein the biomarker is a polypeptide.
6. A method for predicting whether an individual requiring treatment for a disease state involving c-met activity will successfully respond or will not respond to said treatment comprising the steps of: a) obtaining a sample of cells from said individual; b) determining whether said cells express a biomarker of aspect 1; and c) correlating the expression of said marker to the individual's ability to respond to said treatment.
7. The method according to aspect 6 wherein the disease state is cancer.
8. A method of screening for candidate drugs capable of inhibiting the activity of c-met, comprising: a) contacting a test drug with a sample; and b) selecting as candidate drugs those test drugs that modulate activity of a biomarker of aspect 1.

## Claims

1. Use of the expression of VEGF, CD44, Mump-1, or osteopontin by a cell to predict the cell's susceptibility to treatment with a drug that inhibits c-met activity.

2. A method for predicting whether a drug is capable of inhibiting c-met activity in a cell, said method comprising the steps of:
a) obtaining a sample of cells;
b) determining an expression pattern of said sample of cells;
c) determining an expression pattern for at least one control panel of cells, wherein the expression pattern of said control panel includes at least one biomarker whose expression pattern is predictive of a response of cells to treatment with a drug that inhibits c-met activity and provides a predictor expression pattern for cells that are sensitive to or resistant to a drug inhibiting c-met activity in a cell;
d) comparing said polynucleotide expression pattern of said sample cells with the predictor expression pattern; and
e) correlating the polynucleotide expression pattern of said sample of cells to said drug's ability or inability to inhibit c-met activity in said cells.

3. The method according to claim 2, wherein the biomarker is a polynucleotide.

4. The method according to claim 2, wherein the biomarker is a polypeptide expressed by a polynucleotide that provides a predictor expression pattern.

5. A method for predicting whether an individual requiring treatment for a disease state involving c-met activity will respond to said treatment with a c-met modulating drug comprising the steps of:
a) obtaining a sample of cells from said individual, wherein said sample of cells is taken from the diseased tissue;
b) determining an expression profile of said sample of cells from said individual prior to treatment with a c-met modulating drug;
c) determining whether said sample of cells express a biomarker, whose expression pattern is predictive of a response of cells sensitive to treatment with a drug that inhibits c-met activity, by comparing said expression profile of said sample of cells to an expression pattern of at least one predictor expression pattern; and
e) correlating the expression of said marker to the individual's ability to respond to said treatment with said c-met modulating drug.

6. The method according to claim 5, wherein the disease state is cancer.

7. A method of screening for candidate drugs capable of inhibiting the activity of c-met, comprising:
a) providing a host cell containing an expression vector comprising a nucleic acid sequence encoding a c-met biomarker polypeptide;
b) determining the biomarker's biological activity in the absence of a candidate drug;
c) contacting said host cell with said candidate drug;
d) determining said biomarker's biological activity in the presence of said candidate drug;
e) correlating the difference between biological activity of said marker in the presence of said candidate drug and in the absence of said candidate drug to said candidate drugs ability to inhibit c-met activity.

8. A method of monitoring the treatment of an individual having a disease treatable with a drug that modulates c-met activity comprising the steps of
a) taking test cells from said individual;
b) determining an expression pattern of said test cells before and after exposure to a c-met inhibiting drug;
c) comparing said expression patterns of said test cells to predictor expression patterns known to be highly expressed in cells that are sensitive to said c-met inhibiting drug; and
d) continuing treatment of said individual wherein step c) indicates that a correlation remains between said expression patterns.
